# EUROPEAN PATENT APPLICATION

(11) **EP 4 483 896 A2**
(43) Date of publication of application: **01.01.2025**
(21) Application number: 24197275.1
(22) Date of filing: 01.07.2019
(51) Int. Cl.: A61K 39/09

(54) **IMPROVEMENTS IN IMMUNOGENIC CONJUGATES**

(30) Priority: 04.07.2018 US 201862693981 P
(62) Divisional of application: 19745001.8
(71) Applicant: VAXCYTE, INC., San Carlos, California 94070 (US)
(72) Inventor: FAIRMAN, Jeffery, Mountain View, 94040 (US); HEINRICHS, Jon, Doylestown, 18902 (US); CHAN, Wei, San Francisco, 94102 (US)
(74) Representative: Carpmaels & Ransford LLP

(57) **Abstract**

The present application discloses various improvements concerning immunogenic conjugates which comprise a carrier polypeptide and a saccharide antigen, wherein the saccharide antigen is covalently bonded to the carrier polypeptide via a non-natural amino acid residue therein.

## Description

### CROSS-REFERENCE TO RELATED APPLICATIONS

This application priority benefit of provisional U.S. patent application serial number 62/693,981 filed July 4, 2018, the content of which are hereby incorporated by reference in its entirety.

### INCORPORATION OF THE ELECTRONIC TEXT FILE SUBMITTED HEREWITH

The contents of the text file submitted electronically herewith are incorporated herein by reference in their entirety: A computer readable format copy of the Sequence Listing (filename: STRO_005_01WO_SeqList_ST25.txt, date recorded: July 1, 2019, file size ~23 kilobytes).

### BACKGROUND OF THE INVENTION

The immune response to a "weak" saccharide antigen can be amplified by conjugation to a known "strong" carrier polypeptide antigen such as diphtheria toxoid, tetanus toxoid, *H.influenzae* protein D, or CRM197. WO 2018/126229 (SutroVax, Inc., Foster City, California) discloses methods, compositions, and techniques for the production of conjugate vaccine antigens using carrier polypeptides which include a non-natural amino acid (nnAA). Orthogonal attachment chemistry via the nnAA permits conjugation of antigens to the carrier polypeptides to produce immunogenic conjugates which are useful for immunization.

It is an object of the invention to provide variants and improvements of such methods, compositions, and techniques. The variants and improvements described below can be applied to or combined with any of the methods, compositions or techniques disclosed in WO 2018/126229 or in provisional U.S. Patent Applications Serial Nos. 62/693,978 and 62/693,981, both filed 4 July 2018. The aforementioned patent applications are incorporated by reference herein in their entireties.

### SUMMARY OF THE INVENTION

In one embodiment, we provide a sterile container (e.g. a vial) containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein. The container can contain a unit dose of the pharmaceutical composition. Sterile glass containers are preferred.

In another embodiment, we provide a delivery device (*e.g.* syringe, nebulizer, sprayer, inhaler, dermal patch, *etc.)* containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein. The delivery device can contain a unit dose of the pharmaceutical composition. The delivery device can be used to administer the pharmaceutical composition to a mammalian subj ect.

In another embodiment, we provide a hermetically sealed container containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein. Suitable containers for hermetic sealing include *e.g.* a vial. The contents are preferably sterile at the point of hermetic sealing.

In another embodiment, we provide a syringe containing from 0.25-0.75mL (e.g. from 0.3-0.75mL, preferably 0.5mL) of a pharmaceutical composition which comprises two or more different immunogenic conjugates, each comprising a carrier polypeptide and a saccharide antigen, wherein the saccharide antigen is covalently bonded to the carrier polypeptide via a non-natural amino acid residue therein.

In another embodiment, we provide a pharmaceutical composition comprising two or more different immunogenic conjugates and an aluminum salt adjuvant, wherein: (i) each immunogenic conjugate comprises a carrier polypeptide and a saccharide antigen, wherein the saccharide antigen is covalently bonded to the carrier polypeptide via a non-natural amino acid residue therein; (ii) the aluminum salt adjuvant is an aluminum hydroxide or aluminum phosphate adjuvant; <(iii) the pharmaceutical composition has a volume from 0.25-0.75mL *(e.g.* from 0.3-0.75mL, preferably 0.5mL).

In another embodiment, we provide a pharmaceutical composition comprising two or more different immunogenic conjugates, and an aluminum phosphate adjuvant, wherein: (i) each immunogenic conjugate comprises a carrier polypeptide and a saccharide antigen, wherein the saccharide antigen is covalently bonded to the carrier polypeptide via a non-natural amino acid residue therein; and (ii) the concentration of aluminum ions in the composition is <300µg/mL (e.g. betweem 100-300µg/mL). Ideally the concentration of aluminum ions is ≤1.7mg/mL. Conjugates within the composition may be adsorbed to the aluminum phosphate adjuvant.

In another embodiment, we provide a pharmaceutical composition comprising two or more different immunogenic conjugates, and an aluminum phosphate adjuvant, wherein: (i) each immunogenic conjugate comprises a carrier polypeptide and a saccharide antigen, wherein the saccharide antigen is covalently bonded to the carrier polypeptide via a non-natural amino acid residue therein; (ii) the carrier polypeptde does not comprise SEQ ID NO: 3; and (iii) the concentration of aluminum ions in the composition is <2.5mg/mL. Ideally the concentration of aluminum ions is ≤1.7mg/mL. Conjugates within the composition may be adsorbed to the aluminum phosphate adjuvant.

In another embodiment, we provide a pharmaceutical composition comprising two or more different immunogenic conjugates, wherein: (i) each immunogenic conjugate comprises a carrier polypeptide and a saccharide antigen, wherein the saccharide antigen is covalently bonded to the carrier polypeptide via a non-natural amino acid residue therein; and (ii) the volume of the pharmaceutical composition is from 0.25-1.25mL (*e.g.* from 0.3-0.7mL, preferably 0.5mL). This composition may include an aluminum phosphate adjuvant, and conjugates within the composition may be adsorbed to the aluminum phosphate adjuvant.

In another embodiment, we provide a pharmaceutical composition comprising two or more different immunogenic conjugates and a preservative, wherein each immunogenic conjugate comprises a carrier polypeptide and a saccharide antigen, wherein the saccharide antigen is covalently bonded to the carrier polypeptide via a non-natural amino acid residue therein.

In another embodiment, we provide a preservative-free pharmaceutical composition comprising two or more different immunogenic conjugates, wherein each immunogenic conjugate comprises a carrier polypeptide and a saccharide antigen, wherein the saccharide antigen is covalently bonded to the carrier polypeptide via a non-natural amino acid residue therein.

In another embodiment, we provide a pharmaceutical composition comprising two or more different immunogenic conjugates, wherein: (i) each immunogenic conjugate comprises a carrier polypeptide and a saccharide antigen, wherein the saccharide antigen is covalently bonded to the carrier polypeptide via a non-natural amino acid residue therein; (ii) the composition has an osmolality from 200-400 mOsm/kg.

In another embodiment, we provide a pharmaceutical composition comprising two or more different immunogenic conjugates and at least one excipient wherein: (i) each immunogenic conjugate comprises a carrier polypeptide and a saccharide antigen, wherein the saccharide antigen is covalently bonded to the carrier polypeptide via a non-natural amino acid residue therein; and (ii) the at least one excipient is selected from the group consisting of sodium chloride, succinic acid, and polysorbate 80. It may also include an aluminum salt adjuvant. This composition can include both sodium chloride and polysorbate 80 as excipients.

In another embodiment, we provide a pharmaceutical composition comprising *n* different immunogenic conjugates wherein: (i) each of the *n* immunogenic conjugates comprises a carrier polypeptide and a saccharide antigen, wherein the saccharide antigen is covalently bonded to the carrier polypeptide via a non-natural amino acid residue therein; (ii) *n* is an integer from 3 to 50; and (iii) the total amount of carrier polypeptide in the *n* immunogenic conjugates is less than or equal to *3n* µg per dose of the pharmaceutical composition.

In another embodiment, we provide a pharmaceutical composition comprising n different immunogenic conjugates wherein: (i) each of the *n* immunogenic conjugates comprises a carrier polypeptide and a saccharide antigen, wherein the saccharide antigen is covalently bonded to the carrier polypeptide via a non-natural amino acid residue therein; (ii) *n* is an integer from 3 to 50; and (iii) the total concentration of carrier polypeptide in the *n* immunogenic conjugates is less than or equal to *6n* µg/mL in the pharmaceutical composition.

In another embodiment, we provide a pharmaceutical composition comprising *n* different immunogenic conjugates wherein: (i) each of the *n* immunogenic conjugates comprises a carrier polypeptide and a saccharide antigen, wherein the saccharide antigen is covalently bonded to the carrier polypeptide via a non-natural amino acid residue therein; (ii) *n* is an integer from 3 to 50; and (iii) the total amount of saccharide antigen in the *n* immunogenic conjugates is less than or equal to *3n* µg per dose of the pharmaceutical composition.

In another embodiment, we provide a pharmaceutical composition comprising *n* different immunogenic conjugates wherein: (i) each of the *n* immunogenic conjugates comprises a carrier polypeptide and a saccharide antigen, wherein the saccharide antigen is covalently bonded to the carrier polypeptide via a non-natural amino acid residue therein; (ii) *n* is an integer from 3 to 50; and (iii) the total concentration of saccharide antigen in the n immunogenic conjugates is less than or equal to *6n* µg/mL in the pharmaceutical composition.

In another embodiment, we provide a pharmaceutical composition comprising two or more different immunogenic conjugates wherein: (i) each immunogenic conjugate comprises a carrier polypeptide and a saccharide antigen, wherein the saccharide antigen is covalently bonded to the carrier polypeptide via a non-natural amino acid residue therein; and (ii) the average amount of carrier polypeptide per conjugate is from 1-4 µg per dose of the pharmaceutical composition.

In another embodiment, we provide a pharmaceutical composition comprising two or more different immunogenic conjugates wherein: (i) each immunogenic conjugate comprises a carrier polypeptide and a saccharide antigen, wherein the saccharide antigen is covalently bonded to the carrier polypeptide via a non-natural amino acid residue therein; and (ii) the average concentration of carrier polypeptide per conjugate is from 2-8 µg/mL in the pharmaceutical composition.

In another embodiment, we provide a pharmaceutical composition comprising two or more different immunogenic conjugates wherein: (i) each immunogenic conjugate comprises a carrier polypeptide and a saccharide antigen, wherein the saccharide antigen is covalently bonded to the carrier polypeptide via a non-natural amino acid residue therein; and (ii) the average amount of saccharide antigen per conjugate is from 1-4 µg per dose of the pharmaceutical composition

In another embodiment, we provide a pharmaceutical composition comprising two or more different immunogenic conjugates wherein: (i) each immunogenic conjugate comprises a carrier polypeptide and a saccharide antigen, wherein the saccharide antigen is covalently bonded to the carrier polypeptide via a non-natural amino acid residue therein; and (ii) the average concentration of saccharide antigen per conjugate is from 2-8 µg/mL in the pharmaceutical composition.

In another embodiment, we provide a pharmaceutical composition comprising n different immunogenic conjugates wherein: (i) each of the *n* immunogenic conjugates comprises a carrier polypeptide and a saccharide antigen, wherein the saccharide antigen is covalently bonded to the carrier polypeptide via a non-natural amino acid residue therein; (ii) *n* is an integer from 3-50; and either (iii) the composition is free from the carrier polypeptide(s) in unconjugated form or (iv) the composition contains the carrier polypeptide(s) in unconjugated form, wherein the mass of the carrier polypeptide(s) in unconjugated form in the composition is <10% of the mass of that carrier polypeptide in the *n* immunogenic conjugates.

In another embodiment, we provide a pharmaceutical composition comprising *n* different immunogenic conjugates wherein: (i) each of the *n* immunogenic conjugates comprises a carrier polypeptide and a saccharide antigen, wherein the saccharide antigen is covalently bonded to the carrier polypeptide via a non-natural amino acid residue therein; (ii) *n* is an integer from 3-50; and either (iii) the composition is free from the saccharide antigens in unconjugated form or (iv) the composition contains at least one of the saccharide antigens in unconjugated form, wherein the total mass of the saccharide antigens in unconjugated form in the composition is <40% *(e.g.* ≤30%, ≤20%, or ≤10%) of the total mass of the saccharide antigens in the *n* immunogenic conjugates.

In another embodiment, we provide a pharmaceutical composition comprising 14 or more different immunogenic conjugates wherein: (i) each immunogenic conjugate comprises a carrier polypeptide and a saccharide antigen, wherein the saccharide antigen is covalently bonded to the carrier polypeptide via a non-natural amino acid residue therein; and (ii) the total amount of carrier polypeptide per dose is <40µg.

In another embodiment, we provide a pharmaceutical composition comprising 14 or more different immunogenic conjugates wherein: (i) each immunogenic conjugate comprises a carrier polypeptide and a saccharide antigen, wherein the saccharide antigen is covalently bonded to the carrier polypeptide via a non-natural amino acid residue therein; and (ii) the concentration of carrier polypeptide per is ≤80µg/mL.

In another embodiment, we provide a process for preparing a plurality of unit doses of a pharmaceutical composition, wherein (i) the pharmaceutical composition comprises an immunogenic conjugate comprising a carrier polypeptide and a saccharide antigen, wherein the saccharide antigen is covalently bonded to the carrier polypeptide via a non-natural amino acid residue therein, and (ii) the process comprises steps of preparing a bulk composition comprising the immunogenic conjugate and packaging individual unit doses from the bulk composition into a plurality of individual containers. This process is ideally performed aseptically. The individual containers can be sealed after the unit doses have been packaged into them. The individual containers are ideally syringes.

In another embodiment, we provide a pharmaceutical composition comprising two or more different immunogenic conjugates, wherein: (i) each immunogenic conjugate comprises a carrier polypeptide and a saccharide antigen, wherein the saccharide antigen is covalently bonded to the carrier polypeptide via a non-natural amino acid residue therein; (ii) the composition is lyophilized.

In another embodiment, we provide a process for preparing a pharmaceutical composition, wherein the pharmaceutical composition comprises two or more different immunogenic conjugates and an aluminum salt adjuvant, wherein (i) each of the immunogenic conjugates comprises a carrier polypeptide and a saccharide antigen, and (ii) the saccharide antigen is covalently bonded to the carrier polypeptide via a non-natural amino acid residue therein; and the process comprises one of (A) steps of separately adsorbing each of the immunogenic conjugates to an aluminum salt adjuvant then mixing individual adsorbed conjugates together (B) sequentially adsorbing each of the immunogenic conjugates to the aluminum salt adjuvant or (C) preparing a mixture of two or more of the immunogenic conjugates (*e.g.* all of them) and combining this mixture with an aluminum salt adjuvant. The adjuvant may be an aluminum phosphate adjuvant.

In another embodiment, we provide a modified CRM197 carrier polypeptide comprising an amino acid sequence which (i) has at least 80% sequence identity to SEQ ID NO: 1; (ii) is free from an Arg-Arg dipeptide sequence; and (iii) includes at least one nnAA residue. Thus, for instance, Arg-192 and/or Arg-193 of SEQ ID NO: 1 can be deleted or can be substituted with a different amino acid. The nnAA residue(s) can be introduced by substitution of an amino acid residue in SEQ ID NO: 1 and/or by insertion. The modified CRM197 carrier polypeptide can be used to prepare immunogenic conjugates (*e.g.* of saccharide antigens) via the nnAA residue(s) therein.

In another embodiment, we provide a modified CRM197 carrier polypeptide comprising an amino acid sequence which (i) has at least 80% sequence identity to SEQ ID NO: 1 and (ii) includes a nnAA substitution at one or more of the following amino acid residues (numbered according to SEQ ID NO: 1): Asp-211; Asp-295; Asp-352; Asp-392; Asp-465; Asp-467; Asp-507; Asp-519; Asn-296; Asn-359; Asn-399; Asn-481; Asn-486; Asn-502; Asn-524; Glu-240; Glu-248; Glu-249; Glu-256; Glu-259; Glu-292; Glu-362; Gln-252; Gln-287; Lys-212; Lys-218; Lys-221; Lys-229; Lys-236; Lys-264; Lys-299; Lys-385; Lys-456; Lys-474; Lys-498; Lys-516; Lys-522; Lys-534; Arg-377; Arg-407; Arg-455; Arg-460; Arg-462; Arg-472; Arg-493; Ser-198; Ser-200; Ser-231; Ser-233; Ser-239; Ser-261; Ser-374; Ser-381; Ser-297; Ser-397; Ser-451; Ser-475; Ser-494; Ser-495; Ser-496; Ser-501; Ser-505; Thr-253; Thr-265; Thr-267; Thr-269; Thr-293; Thr-386; Thr-400; Thr-408; Thr-469; and/or Thr-517. The modified CRM197 carrier polypeptide can be used to prepare immunogenic conjugates (*e.g.* of saccharide antigens) via the nnAA residue(s) therein.

In another embodiment, we provide a modified CRM197 carrier polypeptide comprising: an amino acid sequence which (i) has at least 80% sequence identity to SEQ ID NO: 1; (ii) is free from an Arg-Arg dipeptide sequence; and (iii) includes a nnAA substitution at one or more of the following amino acid residues (numbered according to SEQ ID NO: 1): Asp-211; Asp-295; Asp-352; Asp-392; Asp-465; Asp-467; Asp-507; Asp-519; Asn-296; Asn-359; Asn-399; Asn-481; Asn-486; Asn-502; Asn-524; Glu-240; Glu-248; Glu-249; Glu-256; Glu-259; Glu-292; Glu-362; Gln-252; Gln-287; Lys-212; Lys-218; Lys-221; Lys-229; Lys-236; Lys-264; Lys-299; Lys-385; Lys-456; Lys-474; Lys-498; Lys-516; Lys-522; Lys-534; Arg-377; Arg-407; Arg-455; Arg-460; Arg-462; Arg-472; Arg-493; Ser-198; Ser-200; Ser-231; Ser-233; Ser-239; Ser-261; Ser-374; Ser-381; Ser-297; Ser-397; Ser-451; Ser-475; Ser-494; Ser-495; Ser-496; Ser-501; Ser-505; Thr-253; Thr-265; Thr-267; Thr-269; Thr-293; Thr-386; Thr-400; Thr-408; Thr-469; and/or Thr-517. The modified CRM197 carrier polypeptide can be used to prepare immunogenic conjugates (*e.g.* of saccharide antigens) via the nnAA residue(s) therein.

In another embodiment, we provide an immunogenic conjugate comprising a carrier polypeptide and a saccharide antigen, wherein (i) the carrier polypeptide comprises amino acid sequence SEQ ID NO: 4; and (ii) the saccharide antigen is covalently bonded to the carrier polypeptide via at least one nnAA residue within SEQ ID NO: 4. We also provide a pharmaceutical composition comprising two or more different immunogenic conjugates which each comprise a carrier polypeptide and a saccharide antigen, wherein (i) the carrier polypeptide in each conjugate comprises amino acid sequence SEQ ID NO: 4; and (ii) the saccharide antigen in each conjugate is covalently bonded to the carrier polypeptide via at least one nnAA residue within SEQ ID NO: 4.

In another embodiment, we provide a syringe containing a pharmaceutical composition which comprises two or more different immunogenic conjugates, each comprising a carrier polypeptide and a pneumococcal saccharide antigen, wherein the syringe is a non-siliconized syringe. The pharmaceutical composition in the non-siliconized syringe ideally has 13 or more different pneumococcal conjugates, and the carrier polypeptides optionally include a nnAA but may instead be *e.g.* CRM197. Further details of non-siliconized syringes are given below.

### BRIEF DESCRIPTION OF THE FIGURE

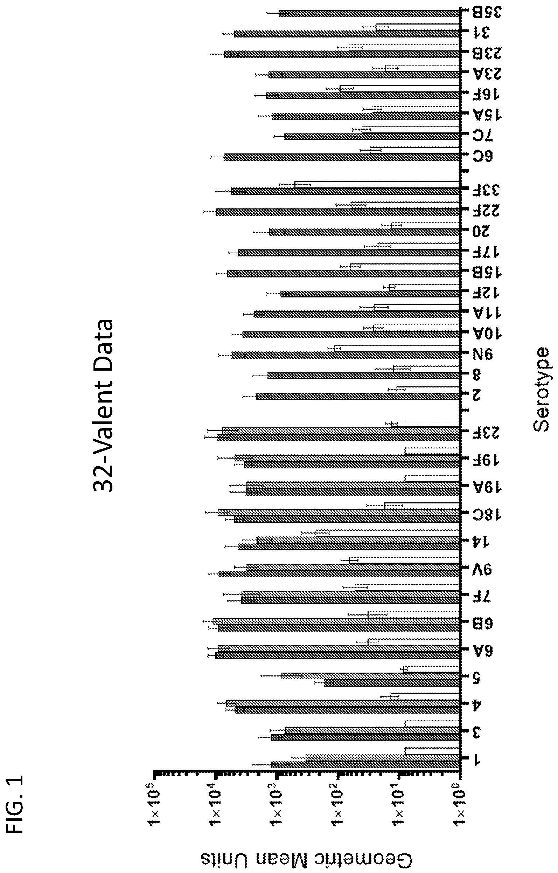

FIG. 1 provides the geometric mean titer for each of the 32 indicated serotypes in a 32-valent vaccine of the present invention, relative to a polysaccharide/alum formulation and Prevnar-13^{™}, as described in the examples.

### DETAILED DESCRIPTION OF THE INVENTION

Various details of methods, compositions, and techniques for the production of conjugated antigens are disclosed in International Patent Publication No. WO2018/126229, the complete contents of which are incorporated herein by reference.

### Immunogenic conjugates

The invention generally concerns immunogenic conjugates. These conjugates comprise a carrier polypeptide which is covalently linked to an antigen. This linking can convert a T-cell independent immunogen (such as a saccharide) into a T-cell dependent immunogen, thereby enhancing the immune response which is elicited (particularly in children). Conjugates used herein include covalent linkages which are formed between an antigen and a non-natural amino acid (`nnAA') residue within the carrier polypeptide. These nnAA residues can provide functional groups which facilitate reactivity with an antigen of interest.

Typically a single carrier polypeptide will be linked to multiple antigen molecules. The antigens can have a single linking group per molecule *(e.g.* the reducing terminus of a saccharide) for attaching to a carrier polypeptide, or can have multiple linking groups (*e.g.* multiple aldehyde or cyanate ester groups). Where an antigen molecule has multiple linking groups this generally leads to the formation of high molecular weight cross-linked or lattice conjugates, involving links between multiple carrier polypeptides via the antigens. Cross-linked conjugates are preferred herein (particularly for pneumococcus), and thus antigens with multiple linking groups are also preferred.

Covalent linkages are formed between the antigen and a nnAA residue within the carrier polypeptide. Preferably the antigen is not conjugated to a lysine residue in the carrier polypeptide; more preferably, the antigen is not conjugated to a natural amino acid residue in the carrier polypeptide.

Useful carrier polypeptides contain a T-cell epitope. Various such carrier polypeptides are known in the art, and within approved vaccines it is known to use diphtheria toxoid (chemically treated toxin from *Corynebacterium diphtheriae;* `Dt'), tetanus toxoid (chemically treated tetanospasmin toxin from *Clostridium tetani;* `Tt'), protein D from *Haemophilus influenzae* ('PD' or `HiD'), the outer membrane protein complex of serogroup B meningococcus (`OMPC'), and the CRM197 mutant *C.diphtheriae* toxin.

A preferred carrier polypeptide upon which to base the carriers of the present invention is CRM197. CRM197 is well-known in the art *(e.g.* see Bröker et al. 2011 Biologicals 39:195-204) and has the following amino acid sequence (SEQ ID NO: 1), where the underlined residue (Glu-52) differs from the natural diphtheria toxin, whereby the substitution of Gly→Glu leads to the loss of toxic enzymatic activity in the protein:

GADDVVDSSKSFVMENFSSYHGTKPGYVDSIQKGIQKPKSGTQGNYDDDWKEFYSTDNKYDAAGYSVDNEN
PLSGKAGGVVKVTYPGLTKVLALKVDNAETIKKELGLSLTEPLMEQVGTEEFIKRFGDGASRVVLSLPFAE
GSSSVEYINNWEQAKALSVELEINFETRGKRGQDAMYEYMAQACAGNRVRRSVGSSLSCINLDWDVIRDKT
KTKIESLKEHGPIKNKMSESPNKTVSEEKAKQYLEEFHQTALEHPELSELKTVTGTNPVFAGANYAAWAVN
VAQVIDSETADNLEKTTAALSILPGIGSVMGIADGAVHHNTEEIVAQSIALSSLMVAQAIPLVGELVDIGF
AAYNFVESIINLFQVVHNSYNRPAYSPGHKTQPFLHDGYAVSWNTVEDSIIRTGFQGESGHDIKITAENTP
LPIAGVLLPTIPGKLDVNKSKTHISVNGRKIRMRCRAIDGDVTFCRPKSPVYVGNGVHANLHVAFHRSSSE
KIHSNEISSDSIGVLGYQKTVDHTKVNSKLSLFFEIKS

The invention does not use native CRM197. Instead of using CRM197 comprising SEQ ID NO: 1, a modified amino acid sequence is used which contains at least one nnAA. These modified CRM197 carrier polypeptides are described in more detail below.

Aside from CRM197, other detoxified mutant forms of diphtheria toxin can be used. For instance, a non-toxic K51E/E148K double mutant has also been used as a carrier polypeptide in conjugates (Pecetta et al. 2016 Vaccine 34:1405-11) and nnAA residues can be incorporated into the sequence of this double mutant in the same way as in CRM197.

Another carrier polypeptide of interest is PD from *H. infZuenzae,* which naturally has the following amino acid sequence (SEQ ID NO: 5):

CSSHSSNMANTQMKSDKIIIAHRGASGYLPEHTLESKALAFAQQADYLEQDLAMTKDGRLVVIHDHFLDGL
TDVAKKFPHRHRKDGRYYVIDFTLKEIQSLEMTENFETKDGKQAQVYPNRFPLWKSHFRIHTFEDEIEFIQ
GLEKSTGKKVGIYPEIKAPWFHHQNGKDIAAETLKVLKKYGYDKKTDMVYLQTFDFNELKRIKTELLPQMG
MDLKLVQLIAYTDWKETQEKDPKGYWVNYNYDWMFKPGAMAEVVKYADGVGPGWYMLVNKEESKPDNIVYT
PLVKELAQYNVEVHPYTVRKDALPEFFTDVNQMYDALLNKSGATGVFTDFPDTGVEFLKGIK

Rather than using native PD, a modified amino acid sequence is used which contains at least one nnAA. For instance, one or more Lys residues within SEQ ID NO: 5 can be replaced with a nnAA. There are 36 Lys residues within SEQ ID NO: 5, so several can be replaced by nnAA and then used for conjugation. T-cell epitope prediction and recognition for PD has been reported by Hua et al. (2016) Clin Vaccine Immunol 23:155-61.

More generally, any polypeptide including a T-cell epitope can be used as a carrier polypeptide. The T-cell epitope can bind to MHC class II and interact with T-cell receptors on the surface of CD4+ T-cells, thereby enhancing antibody responses against antigens or haptens conjugated thereto *(e.g.* see Costantino et al. 2011, Expert Opin Drug Discov 6:1045-66).. Micoli et al. (2018) Molecules 23, 1451 reviews various carrier polypeptides and criteria for their selection. Tontini et al. (2016) Vaccine 34:4235-42 discuss pre-clinical studies of 28 carrier polypeptides, including tests of their ability to induce antibodies against saccharide antigens. Polyepitope carrier polypeptides containing multiple broadly-reactive *(i.e.* immunogenic in the context of most human MHC class II molecules) human CD4+ T-cell epitopes from various pathogen-derived antigens have been designed *e.g.* the N19 and other polypeptides as disclosed by Falugi et al. (2001) Eur J Immunol 31:3816-24, Baraldo et al. (2004) Infect Immun 72:4884-7, and US patents 6,855,321 & 7,867,498. The ability to design these polypepitope carriers demonstrates the ability of those skilled in the art to identify suitable T cell epitopes from diverse sources and also to use them to design effective carrier polypeptides. See also patent application US2016-0101187. T cell epitopes found within known carriers *(e.g.* Tt, PD, CRM197) can be used. Various detoxified bacterial toxins have been successfully used as carriers *e.g.* Tt, Dt, the *P.aeruginosa* exotoxin, the *C.difficile* A & B toxins, *etc.* Many different carrier polypeptides have been used for pneumococcal saccharides *e.g.* CRM197 in Prevnar^{™}, PD, Tt and Dt in Synflorix^{™}, and various peptides in Velasco et al. (1995) Infect Immun 63:961-8. The invention can use any of these numerous carrier polypeptides, modified to include at least one nnAA, to enhance the immunogenicity of antigens of interest.

Carrier polypeptides to be used with the invention, containing nnAA, can be prepared in general using the techniques disclosed in section 6 (`Carrier Protein Production Methods') of WO2018/126229. Preferred carriers contain nnAAs outside of at least one T-cell epitope of the carrier. If the T-cell epitope regions for a carrier are unknown then one can identify the epitopes using standard techniques *e.g.* see Reece et al. (1993) IJ Immunol 151:6175-84, Beissbarth et al. (2005) Bioinformatics 21 Suppl 1: i29-37, Maciel Jr et al. (2008) Virol 378:105-17, Fridman et al. (2012) Oncoimmunol 1:1258-70, *etc.* (including empirical and/or predictive approaches). It is also possible to confirm that any particular modification of a carrier polypeptide's sequence does not eliminate the desired T-cell response to a conjugated antigen, such as the saccharides herein. A preferred group of carriers do not contain any modification, including insertion or substitution of a nnAA, within a T-cell epitope. Particularly preferred carriers contain as least 2, at least 3, at least 4, at least 5, or at least 6 nnAAs. Particularly preferred carriers may also have a maximum of 10, 9, 8, 7 or 6 nnAAs. Particularly preferred ranges of nnAAs in a carrier polypeptide are 2-10, 2-9, 2-8, 2-7, 2-6, 3-10, 3-9, 3-8, 3-7, 3-6, 4-10, 4-9, 4-8, 4-7, and 4-6 nnAAs.

Various antigens can be included within immunogenic conjugates used herein. Typically the antigen is a saccharide. The term "saccharide" includes polysaccharides having 50 or more repeat units, and oligosaccharides having fewer than 50 repeating units. Typically, polysaccharides have from about 50, 55, 60, 65, 70, 75, 80, 85, 90, or 95 repeating units to about 2,000 (sometimes more) repeating units, and optionally from about 100, 150, 200, 250, 300, 350, 400, 500, 600, 700, 800, 900 or 1000 repeating units to about, 1100, 1200, 1300, 1400, 1500, 1600, 1700, 1800, or 1900 repeating units. Oligosaccharides typically have from about 6, 7, 8, 9, or 10 repeating units to about 15, 20, 25, 30, or 35 to about 40 or 45 repeating units.

Useful saccharides for incorporation into immunogenic conjugates include those found in bacteria. These can be non-capsular saccharides (such as an exopolysaccharide e.g. the *S.aureus* exopolysaccharide) but are preferably bacterial capsular saccharides.

Bacterial capsular saccharides are high molecular weight saccharides found in the capsule of Gram-positive or Gram-negative bacteria and they can be used as vaccine antigens. Such capsular saccharides are generally prepared from whole cell lysates or culture supernatant of the corresponding bacterium via processes that involve diafiltration, protein removal, ethanol precipitation, nucleic acid removal, and freeze drying. Bacterial saccharides used with the invention can be intact as found in the bacteria, or can be fragments obtained from intact saccharides *e.g.* obtained by hydrolysis of saccharides purified from the bacteria.

Saccharide antigens of particular interest include, but are not limited to:
- Capsular saccharides of *Spneumoniae.* Further details of pneumococcal capsular saccharides useful as antigens roe implementing the invention are given below.
- Saccharides of *Streptococcus pyogenes:* The antigen can be a saccharide from *S.pyogenes.* In one embodiment the antigen is the capsular saccharide of *S. pyogenes,* which is composed of hyaluronic acid, a high molecular weight polymer where the repeating unit has the structure:
   [→4)-β-D-GlcUA*p*-(1→3)-*β*-D-Glc*p*NAc-(→] which appears to be invariant between *S. pyogenes* serotypes. In another embodiment the antigen is a non-capsular saccharide from *S. pyogenes,* such as the group-A-strep cell wall saccharide, which comprises a backbone of poly-L-rhamnopyranosyl units connected by alternating α-L-(1→3) and α-L-(1→2) linkages, to which N-acetyl-β-D-glucosamine residues are attached at the 3-position of the rhamnose backbone.
- Capsular saccharides of *Streptococcus agalactiae:* The antigen can be a capsular saccharide from *S.agalactiae* (Group B Streptococcus or GBS). There are at least 10 GBS serotypes with distinct capsular saccharide repeating units (Ia, Ib, II-IX), but only a few serotypes are commonly responsible for disease. These include serotypes Ia, Ib, II, III, and V, and conjugates of capsular saccharides from these serotypes can be prepared.
- Capsular saccharides of *Haemophilus influenzae*: The antigen can be a capsular saccharide from *H. influenzae.* There are at least 6 serotypes of *H. influenzae* with distinct capsular saccharide chemical structures (types a-f). However, only type a and type b are considered "high-virulence" strains, and the preferred type *of H. influenzae* capsular saccharide for use with the invention is type b (Hib).
- Capsular saccharides of *Neisseria meningitidis:* The antigen can be a capsular saccharide from *N.meningitidis.* There are at least 13 serogroups of *N.meningitidis* with distinct capsular saccharide chemical structures (serogroups A, B, C, E-29, H, I, K, L, W-135, X, Y, Z, and Z'), but only six (A, B, C, W-135, X, Y) are thought to be life-threatening. The saccharide antigen is usefully derived from any of serogroups A, C, W135, X, or Y.
- Capsular saccharides of *Porphyromonas gingivalis:* The antigen can be a capsular saccharide derived from one of the six serotypes K1, K2, K3, K4, K5 and K6 of *P.gingivalis.*
- Capsular saccharides of *Salmonella typhi:* The antigen can be a Vi saccharide. Vi is the capsular saccharide of *Salmonella typhi* (the *typhi* serovar of *Senterica*)*.* The Vi saccharide is a linear homopolymer of a hexosaminuronic acid, α1,4-*N*-acetylgalactos-aminouronic acid, which is 60 - 90% acetylated at the C-3 position.
- Saccharides of *Staphylococcus aureus:* The antigen can be a saccharide from *S.aureus.* The saccharide can be the exopolysaccharide of *S.aureus,* which is a poly-N-acetylglucosamine (PNAG), or a capsular saccharide of *S.aureus,* which can be *e.g.* serotype 5, serotype 8 or serotype 336.
- Surface saccharides of *Clostridium difficile:* The antigen can be a surface glycan from *C.difficile,* such as PS-I or PS-II.
- Glucans: The antigen can be a glucan containing β-1,3-linkages and/or β-1,6-linkages. These conjugated glucans can be useful for raising an anti-fungal immune response, for example against *Candida albicans.*

Further details of these saccharide antigens can be found in WO2018/126229.

Antigens often do not intrinsically contain functional groups that are suitable or ideal for conjugation. Thus an antigen might need to be functionalized prior to its conjugation to the nnAA. Further details of such functionalization are given below.

### Pneumococcal capsular saccharides

Preferred antigens for use with the invention are capsular saccharides from *Streptococcus pneumoniae. S.pneumoniae* is an encapsulated Gram-positive bacterium that can cause pneumonia, bacteremia, and meningitis. There are at least 90 distinct documented serotypes of *S.pneumoniae* (see *e.g.* Kalin, M. Thorax 1998;53:159-162) which bear capsular saccharides with serotype-specific repeating unit structures. As will be understood by those in the field, it has been proposed that *S.pneumoniae* serotype 20 is actually made up of two closely related serotypes, the capsular polysaacharides of which are largely cross-protecting (Calix et al. 2012 J Biol Chem 287:27885-94). Thus, as would be further appreciated by those of skill in the art, serotype 20 refers to a saccharide that would have previously been classified in the field as serotype 20, and could therefore structurally be either 20A or 20B (from a strain which would have previously been classified in the field as serotype 20, but could genotypically be either 20A or 20B) as disclosed by Calix *et al.* For example, the strain used to produce serotype 20 polysaccharide in Pneumovax^{™} (Merck) is now believed to be serotype 20A. In some instances, 20A may be preferred. In other instances, 20B may be preferred. Prevalence in a target population could be a basis for selecting between these serotypes. Nevertheless, because of strains classified as 20, 20A and 20B are serologically similar, they are largely cross-protective in a vaccine and the choice among strain may not be critical.

The antigen used with the invention can be a capsular saccharide from any of *S.pneumoniae* serotypes 1, 2, 3, 4, 5, 6A, 6B, 7F, 7A, 7B, 7C, 8, 9A, 9L, 9N, 9V, 10F, 10A, 10B, 10C, 11F, 11A, 11B, 11C, 11D, 12F, 12A, 12B, 13, 14, 15F, 15A, 15B, 15C, 16F, 16A, 17F, 17A, 18F, 18A, 18B, 18C, 19F, 19A, 19B, 19C, 20, 21, 22F, 22A, 23F, 23A, 23B, 24F, 24A, 24B, 25F, 25A, 27, 28F, 28A, 29, 31, 32F, 32A, 33F, 33A, 33B, 33C, 33D, 34, 35F, 35A, 35B, 35C, 36, 37, 38, 39, 40, 41F, 41A, 42, 43, 44, 45, 46, 47F, 47A, or 48 (Henrichsen J Clin Microbiol 1995; 33:2759-2762). However, only a subset of these serotypes are commonly responsible for bacterial infection of clinical significance, so the antigen can be a capsular saccharide from any of *S.pneumoniae* serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F. Serotypes 6C, 7C, 15A, 15C, 16F, 20A, 20B, 23A, 23B, 24B, 31, 34, 35B, 35F, 37 and 38 have also become of clinical concern, so the antigen can be a capsular saccharide from one of these *S.pneumoniae* serotypes.

Where the invention uses conjugates from different pneumococcal serotypes, it is preferred to include saccharides from at least 14 different *S.pneumoniae* serotypes *(e.g.* from 15, 16, 17, 18, 19, 20, 21, 22, 23, 24, 25, or more) Where a composition includes 14 or more serotypes, these preferably include the 13 serotypes 1, 3, 4, 5, 6A, 6B, 7F, 9V, 14, 18C, 19A, 19F, and 23F. In addition to these 13 *S.pneumoniae* serotypes a compositions preferably includes one or more of serotypes 2, 8, 9N, 10A, 11A, 12F, 15B, 17F, 20 (alternatively, 20A or 20B), 22F, and/or 33F. Alternatively, in addition to the above 13 serotypes, a composition preferably includes one or more *S.pneumoniae* serotypes 2, 6C, 8, 9N, 10A, 12F, 15A, 15B, 15C, 16F, 17F, 20, 20A, 20B, 22F, 23A, 23B, 24F, 24B, 31, 33F, 34, 35B, 35F and 38. A useful combination of 15 or more (e.g., 16 or more) serotypes includes each of *S.pneumoniae* serotypes 1, 3, 4, 5, 6A, 6B, 7F, 9V, 14, 18C, 19A, 19F, 22F, 23F and 33F, and may also include serotype 8. A useful combination of 20 or more (e.g. 21 or more) *S.pneumoniae* serotypes includes each of serotypes 1, 3, 4, 5, 6A, 6B, 7F, 8, 9V, 10A, 11A, 12F, 14, 15B, 18C, 19A, 19F, 22F, 23F and 33F. A useful combination of 24 or more serotypes includes each of *S.pneumoniae* serotypes 1, 2, 3, 4, 5, 6A, 6B, 7F, 8, 9V, 9N, 10A, 11A, 12F, 14, 15B, 17F, 18C, 19A, 19F, 20, 22F, 23F and 33F.

The structures of common pneumococcal serotype capsular saccharide repeating units are described in Jones *et al.* (Jones C et al. An Acad Bras Cienc. 2005 Jun;77(2):293-324):
Type 1
   [→3)-D-AAT-α-Gal*p*-(1→4)-α-D-Gal*p*A(2/3OAc)-(1→3)-α-D-Gal*p*A-(1→]
Type 2 
[→4)-β-D-Glcp-(1→3)-[α-D-GlcpA-(1→6)-α-D-Glcp-(1→2)]-α-L-Rhap-(1→3)-α-L-
Rhap-(1→3)β-L-Rhap-(1→]
Type 3
   [→3)-β-D-GlcA-(1→4)-β-D-Glc*p*-(1→]
Type 4 
[→3)-β-D-ManpNAc-(1→3)-α-L-FucpNAc-(1→3)-α-D-GalpNAc-(1→4) -α-D-
Galp2,3(S)Py-(1→]
Type 5 
[→4)-β-D-Glcp-(1→4)-[α-L-PnepNAc-(1→2)-β-D-GlcpA-(1→3)] -α-L-FucpNAc-
(1→3)-β-D-Sugp-(1→]
Type 6B
   [→2)-α-D-Galp-(1→3)-α-D-Glcp-(1→3)-α-L-Rhap-(1→4)-D-Rib-ol-(5→P→]
Type 9N 
[→4)-α-D-GlcpA-(1→3)-α-D-Glcp-(1→3)-β-D-ManpNAc-(1→4) -β-D-Glcp-(1→4)-α-
D-GlcpNAc-(1→]
Type 9V 
[→4)-α-D-GlcpA(2/3OAc)-(1→3)-α-D-Galp-(1→3)-β-D-ManpNAc(4/6OAc)-(1→4) -β-
D-Glcp-(1→4)-α-D-Glcp-(1→]
Type 12F 
[→4)-[α-D-Galp-(1→3)]α-L-FucpNAc-(1→3)-β-D-GlcNAc-(1→4)-[α-D-Glc-(1→2) -α-
D-Glc-(1→3)]-β-D-ManNAcA-(→]
Type 14
   [→4)-β-D-Glcp-(1→6)-[β-D-Galp-(1→4)]-β-D-GlcpNAc-(1→3)-β-D-Galp-(1→]
Type 18C 
[→4)-β-D-Glcp-(1→4)-[α-D-Glcp(6OAc) (1→2)][Gro-(1→P→3)]-β-D-Galp-(1→4) -α-
D-Glcp-(1→3)-β-L-Rhap-(1→]
Type 19F
   [→4)-β-D-ManpNAc-(1→4)-α-D-Glcp-(1→2)-α-L-Rhap-(1→P→]
Type 23F

[→4)-β-D-Glcp-(1→4)-[α-L-Rhap-(1→2)]-[Gro-(2→P→3)] -β-D-Galp-(1→4)-β-L-
Rhap-(1→]

A more extensive discussion of the saccharides is found in Geno et al. (2015) Clin. Microbiol. Rev. 28:871-99, in which Table 1 shows the structures for 97 known serotypes. This table also discloses the proportion of saccharide residues which are acetylated when acetylation is not complete.

The capsular saccharide can be O-acetylated. In some embodiments, the capsular saccharide from serotype 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F comprises a saccharide which has a degree of O-acetylation of between 10-100%, between 20-100%, between 30-100%, between 40-100%, between 50-100%, between 60-100%, between 70-100%, between 75-100%, 80-100%, 90-100%, 50-90%, 60-90%, 70-90% or 80-90%. In other embodiments, the degree of O-acetylation is greater than 10%, greater than 20%, greater than 30%, greater than 40%, greater than 50%, greater than 60%, greater than 70%, greater than 80%, greater than 90%, or about 100%. The degree of O-acetylation of the saccharide can determined by proton NMR (see for example Lemercinier & Jones (1996) Carbohydrate Research 296:83-96; Jones et al. (2002) J. Pharmaceutical and Biomedical Analysis 30:1233-1247). Normally the saccharide used to prepare a conjugate will retain at least 50% *(e.g.* 75%, or even 100%) of the O-acetylation levels seen in the starting capsular saccharide purified from a bacterium.

The *S.pneumoniae* capsular saccharides can be obtained directly from bacteria using isolation procedures known to one of ordinary skill in the art (see for example methods disclosed in U.S. Patent App. Pub. Nos. 2006/0228380, 2006/0228381, 2007/0184071, 2007/0184072, 2007/0231340, and 2008/0102498 and WO 2008/118752). As an alternative, they may be obtained from a commercial source (*e.g.,* ATCC).

A pneumococcal capsular saccharide antigen used with the invention can usefully have a molecular weight between 10kDa and 4,000kDa e.g. between 50 kDa and 3,000 kDa, or between 100 kDa and 2,000 kDa. For instance, the molecular weight can be between 100 kDa and 2,000 kDa; between 100 kDa and 1,750 kDa; between 100 kDa and 1,500 kDa; between 100 kDa and 1,250 kDa; between 100 kDa and 1,000 kDa; between 100 kDa and 750 kDa; between 100 kDa and 500 kDa; between 200 kDa and 4,000 kDa; between 200 kDa and 3,500 kDa; between 200 kDa and 3,000 kDa; between 200 kDa and 2,500 kDa; between 200 kDa and 2,000 kDa; between 200 kDa and 2,000 kDa; between 200 kDa and 1,750 kDa; between 200 kDa and 1,500 kDa; between 200 kDa and 1,250 kDa; between 200 kDa and 1,000 kDa; between 200 kDa and 750 kDa; or between 200 kDa and 500 kDa. Further details and guidance with respect to molecular weights are available in U.S. Serial No. 62/693,978, previously incorporated by reference herein.

The capsular saccharide is optionally chemically modified relative to the capsular saccharide found in nature. For example, the saccharide is optionally de-O-acetylated (partially or fully), de-N-acetylated (partially or fully), N-propionated (partially or fully), etc. De-acetylation optionally occurs before, during or after activation, derivatization, or conjugation, but typically occurs before conjugation.

Some embodiments of the invention involve the use of two or more different conjugates. In relation to pneumococcal capsular saccharide conjugates, this means (when using a single type of carrier polypeptide for each conjugate) that each 'different' conjugate has a saccharide from a different pneumococcal serotype.

### Multivalent conjugates

Preferred compositions of the invention involve the use of two or more different conjugates e.g. within a single pharmaceutical composition. These embodiments are also referred to as multivalent. When any two conjugates are described as 'different', or provide different valencies in a `multivalent' composition, this refers to a difference between the combination of carrier polypeptide and antigen in those two conjugates. For example, when a single type of modified CRM197 *(e.g.* SEQ ID NO: 4) is conjugated to a capsular saccharide from a single serotype of pneumococcus, the reaction product will contain many different types of molecule (different molecular weights, different patterns of linkages within each molecule, *etc.),* but are considered as a single conjugate herein. Those of skill in the art are familiar with this heterogeneity at the molecular level and similarly define individual conjugates of a vaccine by the antigen-carrier combination of the particular conjugate, with other properties (such as molecular weight) being an average within the conjugate composition. Two 'different' conjugates have a different carrier polypeptide *(i.e.* having a different amino acid sequence) and/or a different antigen *(i.e.* having a different antigenic structure).

For instance, capsular saccharide antigens may be purified from two different serotypes of pneumococcus. These two different capsular saccharides can be separately conjugated to a carrier polypeptide (which may be the same or different) to provide two different conjugates. Thus, in relation to bacterial capsular saccharide conjugates, the difference between two 'different' conjugates will typically be that one contains capsular saccharide from a first serotype or serogroup of a bacterial species whereas the other contains capsular saccharide from a second serotype or serogroup of that bacterial species *e.g.* capsular saccharides from different serotypes of *Spneumoniae,* or capsular saccharides from different serogroups of *N.meningitidis.* Two conjugates would also be 'different' if they included antigenically distinct capsular saccharides from multiple bacterial species *e.g.* a Hib saccharide conjugate and a meningococcal saccharide conjugate.

Preferred multivalent compositions of the invention include *n* different immunogenic saccharide conjugates, wherein the saccharide antigen in each of the *n* immunogenic conjugates is distinct from the saccharide antigen of the other *n-1* immunogenic conjugates. For instance, if the composition includes antigens from a single bacterial species, there can be capsular saccharides from *n* different serotypes or *n* different serogroups of that species.

This nomenclature in relation to 'different' conjugates is used in the field of conjugate vaccine. For instance, Glesby et al. (2015) J Infect Dis 212:18-27 mentions that the Prevnar^{™} PCV13 vaccine contains `13 different conjugates' because it includes saccharide antigens from 13 different pneumococcal serotypes, separately conjugated to CRM197. Similarly EP-A-2932979 refers to 'an immunogenic composition comprising 13 different polysaccharide-protein conjugates'.

Thus the PCV7 Prevnar^{™} vaccine has 7 different conjugates, the PCV13 Prevnar^{™} vaccine has 13 different conjugates, the Menveo^{™} vaccine has 4 different conjugates, the Menactra^{™} vaccine has 4 different conjugates, the Nimenrix^{™} vaccine has 4 different conjugates, the Menitorix^{™} vaccine has 2 different conjugates, the Menhibrix^{™} vaccine has 3 different conjugates, the Synflorix^{™} vaccine has 10 different conjugates, *etc.*

Multivalent compositions of pneumococcal conjugates preferably include more than 13 different conjugates e.g. 14, 15, 20, 21, 24, 25, or more. Suitable choices of serotypes for these >13-valent compositions are discussed above.

With respect to high valency vaccines (*e.g.,* with greater than 13 different conjugates) it may sometimes be preferred to use more than one carrier polypeptide to reduce the possibility of carrier suppression (*e.g.* see WO98/51339 and WO2011/110241). For example, in a multivalent vaccine comprising *n* different conjugates, a first carrier polypeptide is conjugated to *n-y* different antigens (*e.g.,* the capsular saacharides from different bacterial serotypes or serogroups) and a second polypeptide carrier is conjugated to the remainingy antigens. In a similar manner, three, four or more carriers could be used with the n antigens divided among them. When more than one carrier is used, at least the first carrier is a nnAA-containing carrier polypeptide according to the present invention. In a preferred embodiment, at least the first and second carriers are nnAA-containing carrier polypeptide s according to the present invention

### Non-natural amino acids

As mentioned above, conjugates used herein include covalent linkages between an antigen and a functional group within a nnAA residue in the carrier polypeptide. The side chains of nnAA residues can provide reactive functional groups which are useful for conjugating antigens to discrete sites in the carrier polypeptide.

In general terms the nnAA can be any amino acid that can be incorporated into a polypeptide during translation but is not one of the 20 common amino acids. A nnAA can be conveniently incorporated into a polypeptide by converting a tRNA molecule such that its codon incorporates the nnAA rather than the natural cognate amino acid. One technique for achieving this involves using a "suppression codon" *i.e.* a nucleotide triplet that is introduced into a coding sequence at a desired position and is recognized by a specific tRNA that can recognize a natural stop codon (e.g., an amber, ochre or opal stop codon) but allows translation to continue, with incorporation of the nnAA (thereby suppressing the natural stop codon).

The nnAA residue can be any of the nnAA residues described herein, or any other that has been identified as compatible with cell-based or cell-free protein synthesis (see, *e.g.,* Schultz et al. Annu Rev Biochem. 2010;79:413-44 particularly pp.418-420; and Chin et al. Annu Rev Biochem. 2014;83:5.1-5.30, which are hereby incorporated by reference). Ideally the nnAA is not one which arises naturally within a cell by modification of one of the 20 common amino acids *(e.g.* pyrolysine, selenocysteine, phosphotyrosine, formyl-methionine, *etc.).*

Particularly preferred nnAA for use herein are those which can be incorporated during translation (in a cellular or a cell-free system) with a side chain that provides a functional group which is not found in the side chain of any of the 20 naturally occurring amino acids. Various techniques for incorporating such amino acids into polypeptides are known *e.g.* see Young & Schultz (2010) J Biol Chem 285:11039-44, Maza et al. (2015) Bioconjugate Chem. 26:1884-9, and Zimmerman et al. (2014) Bioconjugate Chem. 25:351-61. WO2018/126229 discloses in detail how nnAA residues can be incorporated into carrier polypeptides *e.g.* using cell-free expression mixtures, orthogonal tRNA/aminoacyl-tRNA synthetase pairs specific for the nnAA, suppression codons, *etc.* See also US patent application US-2017/0267637.

The nnAA can include a chemical group suitable for "click" chemistry reaction with a corresponding group on an antigen of interest. Suitable chemical groups for "click" chemistry include, but are not limited to azido (-N₃), alkyne (-C=C-), alkene (-C=C-) and 1,2,4,5-tetrazine ( 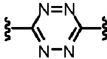 ), and phosphine (*e*.*g*. -P(Ph)₂) groups.

The nnAA can be any of 2-amino-3-(4-azidophenyl)propanoic acid (para-azido-L-phenylalanine or pAF), 2-amino-3-(4-(azidomethyl)phenyl)propanoic acid (para-azidomethyl-L-phenylalanine or pAMF), 2-amino-3-(5-(azidomethyl)pyridin-2-yl)propanoic acid, 2-amino-3-(4-(azidomethyl)pyridin-2-yl)propanoic acid, 2-amino-3-(6-(azidomethyl)pyridin-3-yl)propanoic acid, or 2-amino-5-azidopentanoic acid.

The most preferred nnAA for use herein is pAMF: 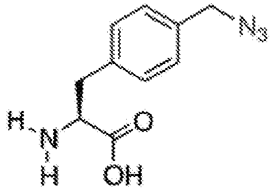 pAMF provides very favorable reaction kinetics for producing conjugates (*e.g.* much faster than using pAF when reacting with an alkyne-containing carbohydrate antigen in a SPAAC method).

The nnAA can be a 2,3-disubstituted propanoic acid bearing: an amino substituent at the 2-position; and an azido-containing substituent, a 1,2,4,5-tetrazinyl-containing substituent, or an ethynyl-containing substituent at the 3-position. Preferably, the substituent at the 3-position is an azido-containing substituent, particularly an azido-containing substituent comprising a terminal azido group bound to the carbon atom at the 3-position through a linking group. For example, the linking group may comprise an arylene moiety that is optionally substituted and optionally heteroatom-containing. For instance, the linking group may comprise a 5- or 6-membered arylene moiety containing 0 to 4 heteroatoms and 0 to 4 non-hydrogen ring substituents.

The nnAA can have the structure of formula XII: 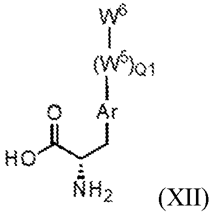 wherein: Ar comprises a 5-membered or 6-membered aromatic ring optionally containing at least one heteroatom; W⁵ is selected from C₁-C₁₀ alkylene, -NH-, -O- and -S-; Q1 is zero or 1; and W⁶ is selected from azido, 1,2,4,5-tetrazinyl optionally C-substituted with a lower alkyl group, and ethynyl. In some embodiments, Ar does not contain any heteroatoms, in which case the preferred linker is an unsubstituted phenylene group *(i.e.* Ar is -C₆H₄-). In other embodiments, Ar contains a nitrogen heteroatom and at least one additional heteroatom selected from N, O, and S. Exemplary nitrogen heterocycles are described *infra* and Ar may be e.g. a pyridine or a pyridazine. In a particularly preferred embodiment, Q1 is 1, W⁵ is lower alkylene, and W⁶ is azido.

The nnAA can be an azido-containing nnAA, such as a nnAA of formula I: 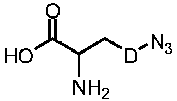 wherein: D is -Ar-W3- or -W 1-Y1-C(O)-Y2-W2-;each of W1, W2, and W3 is independently a single bond or lower alkylene; each X₁ is independently -NH-, -O-, or -S-; each Y1 is independently a single bond, -NH-, or -O-; each Y2 is independently a single bond, -NH-, -O-, or an N-linked or C-linked pyrrolidinylene; Ar is 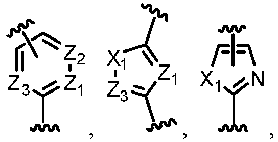 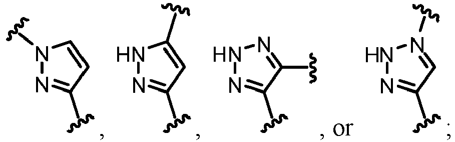 and one of Z₁, Z₂, and Z₃ is -N- and the others of Z₁, Z₂, and Z₃ are independently -CH-.

In other embodiments, the nnAA has formula II: wherein W₄ is C₁-C₁₀ alkylene.

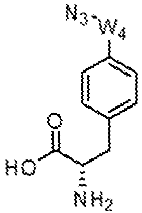

Preparation of azido-containing amino acids according to formulas I and II are found, for example, in Stafford et al. US2014-0066598A1, particularly paragraphs [0331]-[0333], which are incorporated by reference. The process involves substitution of hydroxyl groups for chloride on derivatives of the corresponding aryl amino acids using thionyl chloride, followed by nucleophilic displacement of the chloride with azide. Suitable aryl side-chain containing amino acids are also acquired commercially.

The nnAA can be a 1,2,4,5-tetrazine-containing nnAA. For example, formula III: 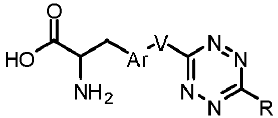 wherein: Ar is 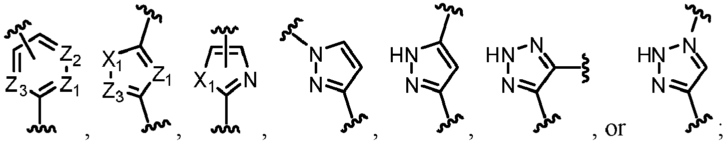 V is a single bond, lower alkylene, or -W1-W2-; one of W1 and W2 is absent or lower alkylene, and the other is -NH-, -O-, or -S-; each one of Z₁, Z₂, and Z₃ is independently -CH- or -N-; and X₁ is independently -NH-, -O-, or -S-; and R is lower alkyl.

Preparation of 1,2,4,5-tetrazine-containing amino acids according to formula III is found, for example, in Yang et al. US2016/0251336, particularly paragraphs [0341]-[0377], which are incorporated by reference. The process involves Negishi coupling of an amino/carboxyl protected derivative of (R)-2-amino-3-iodopropanoic acid with an aminopyridyl bromide to introduce Ar, followed by reaction with a methylthio-1,2,4,5-tetrazine derivative to introduce the tetrazine moiety into the amino acid.

The nnAA can be an alkyne-containing nnAA. In one embodiment, this is a propargyl group. A variety of propargyl-containing amino acids, including syntheses thereof, are found in Beatty et al. Angew. Chem. Int. Ed. 2006, 45, 7364-7; Beatty et al. J. Am. Chem. Soc. 2005(127): 14150-1; Nguyen et al. JACS 2009 (131):8720-1. Such propargyl-containing amino acids are suitable for incorporation into proteins using cell-based systems. In some embodiments, the propargyl-containing nnAA is selected from the group consisting of homopropargylglycine, ethynylphenylalanine, and N6-[(2-propynyloxy)carbonyl]-L-lysine.

The nnAA used herein are generally α-amino acids with a chiral center at the α-carbon, and they are preferably L-stereoisomers.

A polypeptide carrier used with the invention includes at least one nnAA residue. It is preferred that a carrier polypeptide should include multiple nnAA *e.g.* 2, 3, 4, 5, 6, 7, 8, or 9 nnAA residues (or sometimes more). Carrier polypeptides with fewer than 10 nnAA residues are preferred. Thus the polypeptide can include 2-9 nnAA residues, and preferably includes 4-6 nnAA residues.

Where a carrier polypeptide includes multiple nnAA residues it is preferred to include only a single species of nnAA *(e.g.* the only nnAA in the carrier is pAMF). This permits the same conjugation chemistry to be used simultaneously at each nnAA. If it is desired to attach two different antigens to a single carrier molecule, this can be achieved by using different nnAA species within a single carrier and conjugating each antigen to a different nnAA, but conjugation to a single species of nnAA in a carrier is preferred. Moreover, where multiple different conjugates are used (*e.g.* different pneumococcal serotypes) it is sometimes preferred that each conjugate includes the same single species of nnAA. Furthermore, where a composition includes multiple different conjugates (*e.g.* different pneumococcal serotypes) it is sometimes preferred that each conjugate includes the same carrier polypeptide.

A nnAA can be incorporated into a carrier polypeptide by substitution or by insertion (or by C-terminal or N-terminal extension). In one embodiment the nnAA residue(s) are incorporated by substitution. Conveniently, the nnAA can be substituted for a lysine residue in the native polypeptide. For instance, in CRM197 the substitution can occur at one or more of positions K24, K33, K37, K39, K212, K214, K227, K244, K264, K385, K522 and K526 in SEQ ID NO: 1 or 2. Substitution of a nnAA (*e.g.* pAMF) at each of K33, K212, K244, K264, K385, and K526 (and in one embodiment at no other positions) is preferred.

Substitutions to incorporate nnAA are not limited to lysine positions, however, and it is also possible to substitute other amino acids with a nnAA *e.g.* Phe, Asp, Asn, Glu, Gln, Arg, Ser, and/or Thr.

The nnAA within a carrier polypeptide is/are ideally surface-accessible residues. This can be assessed using a 3D structure of the polypeptide, or by performing a comprehensive replacement of natural amino acids for nnAAs followed by conjugation tests, to assess the utility of each site.

To preserve the function of a carrier polypeptide it preferred not to incorporate a nnAA within a T-cell activating epitope of the carrier polypeptide. The use of nnAA allows the selective placement of sites for conjugation and so the T-cell activating epitopes of a carrier polypeptide can be avoided as sites for antigen conjugation. As mentioned above, these epitopes can easily be identified. For example, studies of CRM197 by Raju *et al.,* Bixler *et al.,* Leonard *et al.,* and Pillai *et al.* (*e.g.* Eur J Immunol. 1995 Dec;25(12):3207-14, WO89/06974) have identified various T-cell epitopes e.g. within residues P271-D290, V321-G383, and Q411-I457. Thus it is preferred to avoid introducing nnAA within these regions of SEQ ID NO: 1.

### Conjugation

Conjugation involves formation of a covalent linkage between the nnAA residue and the antigen. This requires a reactive functional group in both the nnAA and the antigen. A nnAA for the carrier polypeptide will generally be chosen because it already has a suitable functional group *(e.g.* the azido group of pAMF), but antigens often do not intrinsically contain functional groups that are suitable or ideal for conjugation. Thus an antigen might need to be functionalised prior to its conjugation to the nnAA.

Detailed technical information about conjugation can be found in Bioconjugate Techniques (Greg T Hermanson, 3rd edition, 2013). WO2018/126229 discloses in detail how antigens can be functionalised and then conjugated to nnAA. As noted above, useful nnAA include a functional group *(e.g.* an azido group) which is suitable for a "click" chemistry reaction with a functional group on the antigen. Thus a functionalised antigen ideally includes a group suitable for such "click" reactions.

In general terms, conjugation thus takes place by a process comprising 3 steps: (a) activating the antigen; (b) optionally derivatizing the activated antigen (*e.g.* with a linker or nucleophilic group) to introduce a reactive functional group not normally present in the antigen; and (c) conjugating the antigen to the carrier polypeptide via a group introduced in step (a) or, if present, step (b). In some embodiments step (a) includes a first step of removing a blocking group on the antigen, such that certain functional groups (*e.g.* hydroxyls, amines, thiols) are more accessible to activation. Sometimes the steps (a)-(c) can occur essentially simultaneously (*e.g.* where a reactive moiety such as N-hydroxysuccinimide is added to the antigen), but in other embodiments two or more of steps (a)-(c) are discrete, with optional purification between steps.

As noted above, cross-linked conjugates are preferred, so it is also preferred to introduce multiple reactive functional groups per antigen molecule. For instance, multiple aldehyde or cyanate ester groups can be introduced when activating a saccharide molecule. These groups can then be derivatised *e.g.* to introduce a reactive cyclooctyne which can then react with azido groups in in the nnAA.

An antigen can be activated using various chemistry, including but not limited to: periodate oxidation (*e.g.* to oxidize hydroxyl groups on adjacent carbon atoms to give reactive aldehyde groups), for instance as disclosed in WO2011/110531; cyanylation *e.g.* using 1-cyano-4-dimethylamino pyridinium tetrafluoroborate (CDAP); hydroxyl activation with 1,1'-carbonyldiimidazole (CDI) followed by nucleophilic addition; or unmasking of an intrinsic aldehyde *(e.g.* a reducing terminus of a saccharide).

Periodate oxidation and CDAP cyanylation are two preferred activation techniques. Periodate oxidation has been shown to be useful for activating *inter alia* pneumococcal serotypes 1, 2, 3, 7F, 8, 9N, and 11A. CDAP cyanylation has been shown to be useful for activating *inter alia* pneumococcal serotypes 3, 7F, and 10A.

The activated antigen can be conjugated to a nnAA directly, but usually the activated group is derivatized to introduce a functional group which shows better reactivity towards the nnAA's functional group. For instance, an alkynyl group can be introduced. A bifunctional reagent with an amino group and an alkyne group can react with an aldehyde group which has been introduced into an antigen (*e.g.* via reductive amination) thereby leaving a pendant alkyne which can react with a nnAA. For instance, bifunctional reagents including amino and DBCO functional groups can be used.

In one embodiment, the nnAA reacts with an alkynyl group in the antigen *(e.g.* a propargyl group). An alkyne group in an antigen is ideal for reacting with an azido group in a nnAA *e.g.* using the reactions known in the art as copper-catalyzed azide-alkyne cycloaddition (CuAAC), ruthenium-catalyzed azide-alkyne cycloaddition (RuAAC), or Huisgen azide-alkyne 1,3-dipolar cycloaddition. The alkynyl group may have a molecular context that increases its reactivity *e.g.* it can be within a ring. For instance, the alkylene can be within a cyclooctyne ring (optionally including a heteroatom), such as a diaryl-strained cyclooctyne ring *(e.g.* DBCO). This reaction can be a [3+2] cycloaddition referred to in the art as strain-promoted azide-alkyne cycloaddition (SPAAC). DIFO- and DBCO-based reagents are readily available for these reactions.

Alkyne-containing rings useful in SPAAC reactions include difluorinated cyclooctyne (DIFO) and dibenzocyclooctynes. These are available with pendant functional groups for linking to activated antigens (*e.g.* with a pendant amino for linking to an aldehyde or a cyanate ester), for instance using any of the following reagents:

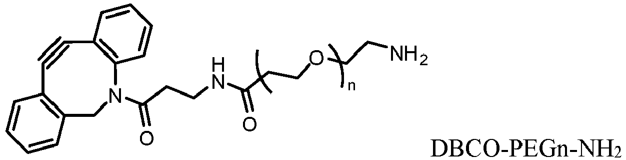

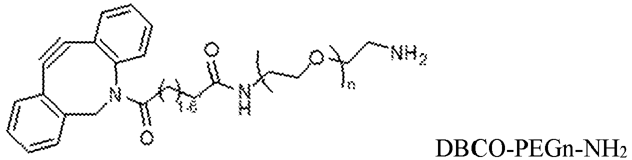

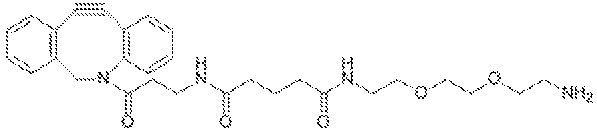

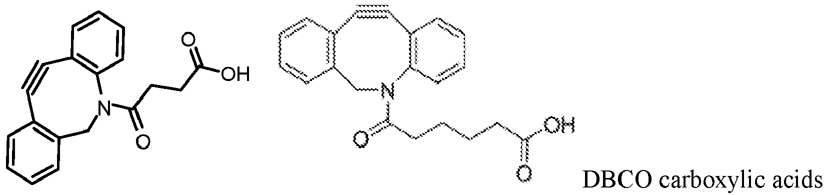

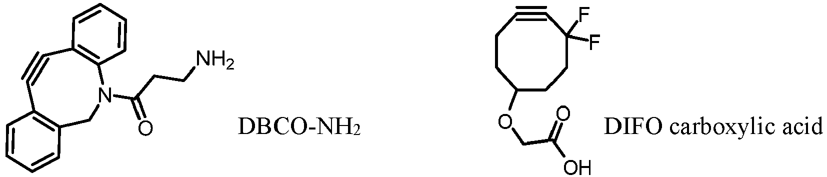

The value of 'η' in 'PEGn' represents the number of oxyethylene repeat units. The value of n is in the range 1-20 *e.g.* within 2-18, 3-16, or 4-14. Thus n can be, for example, any of 4, 5, 11, 12 or 13.

Other click chemistry reactions which can be used for conjugation of an antigen and a nnAA include, but are not limited to, tetrazine-alkene ligation and Staudinger ligation between a phosphine and an azide.

A conjugate of the invention can have a molecular weight of at least about 750 kDa, at least about 1,000 kDa, or at least about 1,500 kDa, or more. In some embodiments, the conjugate has a molecular weight of between about 750 kDa and about 5,000 kDa. In some embodiments, the conjugate has a molecular weight of between about 800 kDa and about 2,800 kDa. In some embodiments, the conjugate has a molecular weight of between about 850 kDa and about 2,800 kDa. In some embodiments, the conjugate has a molecular weight of between about 900 kDa and about 2,800 kDa. In some embodiments, the conjugate has a molecular weight of between about 950 kDa and about 2,800 kDa. In some embodiments, the conjugate has a molecular weight of between about 1,000 kDa and about 2,800 kDa. The molecular weight of a conjugate is calculated by size exclusion chromatography (SEC) combined with multiangle laser light scattering (MALS).

Conjugates of the invention include antigen (*e.g.,* saccharide) and carrier polypeptide, and the weight ratio of these two components can be used as a parameter to define the conjugate. Higher antigen: carrier weight ratios for saccharide-carrier conjugates allow for more saccharide antigen to be delivered with a lower amount of carrier polypeptide. For pneumococcal conjugate vaccines, the ratio is typically in the range 0.3-3.0, but this can vary with the serotype and aspects of the conjugation chemistry (Annex 2: Recommendations for the production and control of pneumococcal conjugate vaccines; WHO Technical Report Series, No. 927, 2005). The ratio of the commercial vaccine Prevnar-13^{™} is 0.9. For compositions which include conjugates of multiple pneumococcal serotypes (*e.g.* more than 13 serotypes) the ratio for the complete composition is ideally above 1.0 *(i.e.* a weight excess of pneumococcal saccharide antigen) and is preferably 1.5 or more (*e.g.* within the range 1.5-3.0, or preferably 1.5-2.0).

### Modified CRM197 carrier polypeptides

As mentioned above, the carrier polypeptides of main interest herein are modified forms of CRM197. Thus preferred carrier polypeptides for use with the invention comprise an amino acid sequence which has at least 80% sequence identity (*e.g.* ≥85%, ≥90%, ≥95%, ≥96%, ≥97%, or preferably ≥98%) to SEQ ID NO: 1. For instance, the carrier polypeptide can comprise the amino acid sequence SEQ ID NO: 1 except for the presence of up to 10 nnAA, as discussed above.

SEQ ID NO: 1 includes an Arg-Arg dipeptide sequence at positions 192-193. This sequence can be subject to proteolytic cleavage in some circumstances. If desired, this site can be modified to prevent cleavage and improve yield. Thus in some embodiments a modified CRM197 carrier polypeptide used herein is free from an Arg-Arg dipeptide sequence. For instance, Arg-192 and/or Arg-193 of SEQ ID NO: 1 can be deleted or can be substituted with a different amino acid. Thus a preferred carrier polypeptide comprises an amino acid sequence which (i) has at least 80% *(e.g.* ≥85%, ≥90%, ≥95%, ≥96%, ≥97%, or preferably ≥98%) sequence identity to SEQ ID NO: 1; (ii) is free from an Arg-Arg dipeptide sequence; and (iii) includes at least one *(e.g.* at least 2, and preferably more, as discussed above) nnAA residue.

One such amino acid sequence is SEQ ID NO: 2, which differs from SEQ ID NO: 1 by having an Arg→Asn substitution at position 193:

GADDVVDSSKSFVMENFSSYHGTKPGYVDSIQKGIQKPKSGTQGNYDDDWKEFYSTDNKYDAAGY
SVDNENPLSGKAGGVVKVTYPGLTKVLALKVDNAETIKKELGLSLTEPLMEQVGTEEFIKRFGDG
ASRVVLSLPFAEGSSSVEYINNWEQAKALSVELEINFETRGKRGQDAMYEYMAQACAGNRVRNSV
GSSLSCINLDWDVIRDKTKTKIESLKEHGPIKNKMSESPNKTVSEEKAKQYLEEFHQTALEHPEL
SELKTVTGTNPVFAGANYAAWAVNVAQVIDSETADNLEKTTAALSILPGIGSVMGIADGAVHHNT
EEIVAQSIALSSLMVAQAIPLVGELVDIGFAAYNFVESIINLFQVVHNSYNRPAYSPGHKTQPFL
HDGYAVSWNTVEDSIIRTGFQGESGHDIKITAENTPLPIAGVLLPTIPGKLDVNKSKTHISVNGR
KIRMRCRAIDGDVTFCRPKSPVYVGNGVHANLHVAFHRSSSEKIHSNEISSDSIGVLGYQKTVDH
TKVNSKLSLFFEIKS

Any embodiment described herein, or in WO2018/126229, by reference to SEQ ID NO: 1 can be put into effect using SEQ ID NO: 2 instead.

Thus we provide a carrier polypeptide comprising amino acid sequence SEQ ID NO: 2, wherein SEQ ID NO: 2 has been modified to include from 1-10 *(e.g.* from 3-9 or from 2-8, or from 2-6, or from 3-6, or from 4-6) nnAA residues. These nnAA residue modifications can be incorporated into SEQ ID NO: 2 as insertions and/or substitutions (*e.g.* SEQ ID NO: 4, which includes 6 Lys→nnAA substitutions). Residue Asn-193 of SEQ ID NO: 2 is preferably not substituted by a nnAA. This carrier polypeptide can be used to prepare immunogenic conjugates *(e.g.* of saccharide antigens) via the nnAA residue(s) therein.

In some embodiments these carrier polypeptides include amino acid sequences upstream and/or downstream of SEQ ID NO: 1 or 2. Thus, for instance, they can include a methionine residue upstream of the N-terminus amino acid residue of SEQ ID NO: 1 or 2. This methionine residue may be formylated. A methionine residue is not present at this position in wild-type CRM197 but it can be included herein for initiating translation (*e.g.* in a cell-free polypeptide synthesis system) without requiring the whole native leader sequence. In some embodiments a carrier polypeptide includes (i) no amino acids upstream of the N-terminus of SEQ ID NO: 1 or 2, except for an optional methionine, and (ii) no amino acids downstream of the C-terminus of SEQ ID NO: 1 or 2.

Preferably, at least one Lys residue in SEQ ID NO: 1 or 2 is substituted by a nnAA residue. It is preferred to substitute more than one residue in SEQ ID NO: 1 or 2 with a nnAA and, ideally, only one species of residue in SEQ ID NO: 1 is substituted by a nnAA *e.g.* only Lys residues are substituted. Where more than one residue in SEQ ID NO: 1 is substituted for a nnAA it is preferred that the same nnAA is used at each position *e.g.* pAMF at each substitution position. As noted above, in some embodiments residues other than Lys are substituted.

Carrier polypeptides comprising amino acid sequence SEQ ID NO: 1 or 2 with from 2-9 substitutions by nnAA residues (*e.g.* Lys→nnAA substitutions, preferably Lys→pAMF) are preferred, and ideally with from 2-8, 2-6, 3-8, 3-6, 4-9, 4-8, or 4-6 nnAA substitutions *e.g.* 4, 5 or 6 nnAA residues. This permits more extensive attachment of antigens to the carrier than using a single nnAA, thereby increasing the antigen: carrier ratio, while avoiding excessive disruption of the native sequence and structure, which can result in insolubility.

Structural studies of CRM197 reveal two general 3D regions within SEQ ID NO: 1 or 2: the first region runs from the N-terminus to Asn-373; and the second region runs from Ser-374 to the C-terminus. These first of these corresponds roughly to the domains known as 'C' & 'T' (catalytic and transmembrane), and the second to domain 'R' (receptor binding). Ideally a carrier polypeptide includes at least one nnAA in the first region and at least one nnAA in the second region *e.g.* at least 2 nnAA in each region, or at least 3 nnAA in each region. This permits conjugated antigens to be spatially separated when attached to the carrier. A carrier with 3 nnAA in the first region and 3 nnAA in the second region is useful.

The first region contains 27 Lys residues, and the second region contains 12 Lys residues. Thus one or more *(e.g.* 3) Lys residues within the N-terminal 374 amino acids and one or more *(e.g.* 3) Lys residues within the C-terminal 162 amino acids of SEQ ID NO: 1 or 2 can be substituted with a nnAA *e.g.* within pAMF.

Preferred embodiments of nnAA-containing carriers based on CRM197 have the amino acid sequence of SEQ ID NO: 1 or SEQ ID NO: 2 in which one or more of residues K24, K33, K37, K39, K212, K214, K227, K264, K385, K522 and K526 is/are replaced by a nnAA (such as pAMF). One such sequence is SEQ ID NO:3, in which each X represents a nnAA (preferably the same nnAA, such as pAMF):

MGADDVVDSSKSFVMENFSSYHGTKPGYVDSIQXGIQKPKSGTQGNYDDDWKEFYSTDNKYDAAG
YSVDNENPLSGKAGGVVKVTYPGLTKVLALKVDNAETIKKELGLSLTEPLMEQVGTEEFIKRFGD
GASRVVLSLPFAEGSSSVEYINNWEQAKALSVELEINFETRGKRGQDAMYEYMAQACAGNRVRRS
VGSSLSCINLDWDVIRDXTKTKIESLKEHGPIKNKMSESPNKTVSEEKAXQYLEEFHQTALEHPE
LSELXTVTGTNPVFAGANYAAWAVNVAQVIDSETADNLEKTTAALSILPGIGSVMGIADGAVHHN
TEEIVAQSIALSSLMVAQAIPLVGELVDIGFAAYNFVESIINLFQVVHNSYNRPAYSPGHXTQPF
LHDGYAVSWNTVEDSIIRTGFQGESGHDIKITAENTPLPIAGVLLPTIPGKLDVNKSKTHISVNG
RKIRMRCRAIDGDVTFCRPKSPVYVGNGVHANLHVAFHRSSSEKIHSNEISSDSIGVLGYQKTVD
HTKVNSXLSLFFEIKS (SEQ ID NO: 3)

Another such sequence is SEQ ID NO: 4, in which each X represents a nnAA (preferably the same nnAA, such as pAMF):

MGADDVVDSSKSFVMENFSSYHGTKPGYVDSIQXGIQKPKSGTQGNYDDDWKEFYSTDNKYDAAG
YSVDNENPLSGKAGGVVKVTYPGLTKVLALKVDNAETIKKELGLSLTEPLMEQVGTEEFIKRFGD
GASRVVLSLPFAEGSSSVEYINNWEQAKALSVELEINFETRGKRGQDAMYEYMAQACAGNRVRNS
VGSSLSCINLDWDVIRDXTKTKIESLKEHGPIKNKMSESPNKTVSEEKAXQYLEEFHQTALEHPE
LSELXTVTGTNPVFAGANYAAWAVNVAQVIDSETADNLEKTTAALSILPGIGSVMGIADGAVHHN
TEEIVAQSIALSSLMVAQAIPLVGELVDIGFAAYNFVESIINLFQVVHNSYNRPAYSPGHXTQPF
LHDGYAVSWNTVEDSIIRTGFQGESGHDIKITAENTPLPIAGVLLPTIPGKLDVNKSKTHISVNG
RKIRMRCRAIDGDVTFCRPKSPVYVGNGVHANLHVAFHRSSSEKIHSNEISSDSIGVLGYQKTVD
HTKVNSXLSLFFEIKS (SEQ ID NO: 4)

SEQ ID NOs: 3 & 4 can be very well-expressed in a cell-free protein synthesis system, while retaining good solubility and providing good immunogenic responses when conjugated to pneumococcal capsular saccharides. SEQ ID NO: 4 lacks the native Arg-Arg dipeptide.

A polypeptide consisting of SEQ ID NO: 4, in which each X is pAMF, is another preferred carrier polypeptide for use with the invention.

WO2018/126229 describes several amino acid residues which are suitable for nnAA substitution (e.g. Lys-24, Lys-33, Lys-37, Lys-39, Lys-212, Lys-214, Lys-227, Lys-244, Lys-264, Lys-385, Lys-522, Lys-526, Phe-12, Phe-53, Phe-123, Phe-127, Phe-140, Phe-167, Phe-250, Phe-389, Phe-530, or Phe-531, numbered according to SEQ ID NO: 1 herein). Other residues which can be substituted are: Asp-211; Asp-295; Asp-352; Asp-392; Asp-465; Asp-467; Asp-507; Asp-519; Asn-296; Asn-359; Asn-399; Asn-481; Asn-486; Asn-502; Asn-524; Glu-240; Glu-248; Glu-249; Glu-256; Glu-259; Glu-292; Glu-362; Gln-252; Gln-287; Lys-212; Lys-218; Lys-221; Lys-229; Lys-236; Lys-264; Lys-299; Lys-385; Lys-456; Lys-474; Lys-498; Lys-516; Lys-522; Lys-534; Arg-377; Arg-407; Arg-455; Arg-460; Arg-462; Arg-472; Arg-493; Ser-198; Ser-200; Ser-231; Ser-233; Ser-239; Ser-261; Ser-374; Ser-381; Ser-297; Ser-397; Ser-451; Ser-475; Ser-494; Ser-495; Ser-496; Ser-501; Ser-505; Thr-253; Thr-265; Thr-267; Thr-269; Thr-293; Thr-386; Thr-400; Thr-408; Thr-469; and/or Thr-517.

We also provide a polypeptide comprising an amino acid sequence which (i) has at least 80% *(e.g.* ≥85%, ≥90%, ≥95%, ≥96%, ≥97%, or preferably ≥98%) sequence identity to SEQ ID NO: 1; (ii) is free from an Arg-Arg dipeptide sequence; and (iii) includes at least one nnAA residue; and wherein the polypeptide has a N-terminus methionine and/or is in monomeric form.

These CRM197-derived carrier polypeptides can be used in the same manner for conjugation as CRM197 has been used in the prior art *(e.g.* see Bröker *et al.* 2011 *supra,* WO2015/117093, *etc.),* but with the improvement of permitting site-specific conjugation via the nnAA residue(s). They will generally be used in monomeric monomeric form, rather than being associated with other CRM197 or CRM197-derived subunits to form polypeptide multimers. Similarly, they will generally include at least one disulfide bridge *e.g.* between Cys-186 & Cys-201 (numbered according to SEQ ID NO: 1) and, optionally, between Cys-461 & Cys-471.

We also provide an immunogenic conjugate comprising any of these various carrier polypeptides conjugated to a saccharide antigen via at least one of its nnAA. The carrier polypeptides are particularly useful for conjugating to pneumococcal capsular saccharides via the nnAA residue(s) therein. Immunogenic conjugates prepared in this way can be combined to form multivalent compositions as discussed elsewhere herein.

Thus we provide an immunogenic conjugate comprising a carrier polypeptide and a saccharide antigen, wherein (i) the carrier polypeptide has amino acid sequence SEQ ID NO: 4 *e.g.* where each X is pAMF; and (ii) the saccharide antigen is covalently bonded to the carrier polypeptide via at least one nnAA residue within SEQ ID NO: 4. We also provide a multivalent pharmaceutical composition comprising two or much such immunogenic conjugates.

Thus we provide a pharmaceutical composition including multiple different conjugates *(e.g.* different pneumococcal serotypes) in which each conjugate includes a carrier polypeptide having amino acid sequence SEQ ID NO: 4.

We also provide an immunogenic conjugate comprising a carrier polypeptide and a saccharide antigen, wherein (i) the carrier polypeptide has amino acid sequence SEQ ID NO: 4; (ii) the saccharide antigen is covalently bonded to the carrier polypeptide via at least one nnAA residue within SEQ ID NO: 4; and (iii) the saccharide antigen is a capsular saccharide from any of pneumococcal serotypes 1, 2, 3, 4, 5, 6A, 6B, 7F, 8, 9N, 9V, 10A, 11A, 12F, 14, 15B, 17F, 18C, 19A, 19F, 20, 22F, 23F and 33F. These individual conjugates can be combined to make multivalent pharmaceutical compositions of the invention.

We also provide a polynucleotide encoding a carrier polypeptide described herein. In another embodiment, the disclosure provides for an expression vector comprising the polynucleotide. In another embodiment, the disclosure provides for a host cell comprising the expression vector.

### Adjuvants

Pharmaceutical compositions of the invention can include an aluminum salt adjuvant. The adjuvant can enhance the immunogenicity of conjugates within the pharmaceutical composition. Conjugates within the composition may be adsorbed to the aluminum salt adjuvant.

Useful aluminum salt adjuvants include, but are not limited to, aluminum hydroxide adjuvants and aluminum phosphate adjuvants. These adjuvants are described *e.g.* in chapters 8 & 9 of Vaccine Design... (1995) eds. Powell & Newman. ISBN: 030644867X. Plenum.

The adjuvants commonly known as "aluminum hydroxide" are typically aluminum oxyhydroxide salts, which are usually at least partially crystalline. Aluminum oxyhydroxide, which can be represented by the formula AlO(OH), can be distinguished from other aluminum compounds, such as Al(OH)₃, by infrared (IR) spectroscopy, in particular by the presence of an adsorption band at 1070cm⁻¹ and a strong shoulder at 3090-3100cm⁻¹ (chapter 9 of Powell & Newman). The degree of crystallinity of an aluminum hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g.* as seen in transmission electron micrographs) is typical for aluminum hydroxide adjuvants *e.g.* with needle-like particles with diameters about 2nm. The pI of aluminum hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminum hydroxide adjuvants.

The adjuvants commonly known as "aluminum phosphate" are typically aluminum hydroxyphosphates, often also containing a small amount of sulfate *(i.e.* aluminum hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e*.*g*. when heated to 200°C) indicates the presence of structural hydroxyls (chapter 9 of Powell & Newman).

The PO₄/Al³⁺ molar ratio of an aluminum phosphate adjuvant will generally be between 0.3 and 1.2, preferably between 0.8 and 1.2, and more preferably 0.95+0.1. The aluminum phosphate will generally be amorphous, particularly for hydroxyphosphate salts. A typical adjuvant is amorphous aluminum hydroxyphosphate with PO₄/Al molar ratio between 0.84 and 0.92, included at 0.6mg Al³⁺/ml. The aluminum phosphate will generally be particulate *(e.g.* plate-like morphology as seen in transmission electron micrographs, with primary particles in the range of 50nm). Typical diameters of the particles are in the range 0.5-20µm (*e.g.* about 5-10µm) after any antigen adsorption. Adsorptive capacities of between 0.7-1.5 mg protein per mg Al⁺⁺⁺ at pH 7.4 have been reported for aluminum phosphate adjuvants.

The point of zero charge (PZC) of aluminum phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminum phosphates used according to the invention will generally have a PZC of between 4.0 and 7.0, more preferably between 5.0 and 6.5 *e.g.* about 5.7.

The concentration of aluminum ions in a composition for administration to a patient is preferably less than 2.5mg/ml *e.g.* ≤2 mg/ml, ≤1 mg/ml, *etc.* A preferred maximum concentration is ≤1.7mg/mL. The range of Al⁺⁺⁺ in a composition of the invention can be from 0.3-1mg/ml or from 0.3-0.5mg/ml. A maximum of 0.85mg/dose is preferred.

In solution both aluminum phosphate and aluminum hydroxide adjuvants tend to form stable porous aggregates 1-10µm in diameter. A composition can include a mixture of both an aluminum hydroxide adjuvant and an aluminum phosphate adjuvant.

Where a composition includes multiple conjugates and each of these is adsorbed to an aluminum salt adjuvant, each conjugate can be adsorbed to an aluminum salt individually and then mixed, or they can each be added in sequence to an aluminum salt, thereby forming the mixed conjugate composition. A mixture of both approaches can also be used.

### Excipients for pharmaceutical compositions

Pharmaceutical compositions of the invention will generally include one or more pharmaceutically acceptable excipient(s). A comprehensive discussion of such excipients can be found in Handbook of Pharmaceutical Excipients (ed. Rowe et al.), 6th edition 2009.

Pharmaceutical compositions are preferably in aqueous form, particularly at the point of administration, but they can also be presented in dried forms *(e.g.* as lyophilisates, *etc.)* which can be converted into aqueous forms for administration.

Pharmaceutical compositions can include a buffer or pH adjusting agent. The buffer can be selected from the group consisting of a phosphate buffer, an acetate buffer, a histidine buffer, a citrate buffer, a succinate buffer, a Tris buffer, a HEPES buffer, *etc.* Buffer salts will typically be included in the 5-20mM range.

Pharmaceutical compositions can include a physiological salt, such as a sodium salt *e.g.* to control tonicity. Sodium chloride (NaCl) is typical, which may be present at from 1-20 mg/ml *e.g.* 10±2 mg/ml or 9 mg/ml. Other salts that may be present include potassium chloride, potassium dihydrogen phosphate, disodium phosphate dehydrate, magnesium chloride, calcium chloride, *etc.* Other useful salts may have sodium, potassium or ammonium cations and chloride, citrate, ascorbate, borate, phosphate, bicarbonate, sulfate, thiosulfate or bisulfite anions.

Pharmaceutical compositions can include an organic acid, such as acetic acid or succinic acid. This may be part of a buffer system.

Pharmaceutical compositions can include a sugar alcohol such as mannitol or sorbitol. Pharmaceutical compositions can include a sugar such as sucrose or glucose.

Pharmaceutical compositions can include a surfactant. Suitable surfactants include, but are not limited to, polysorbate 20, polysorbate 80, and sodium dodecyl sulfate (SDS). In some embodiments the surface active agent is present at a concentration from 0.0003% and 0.3% (w/w) *e.g.* from 0.01-0.03%. Polysorbate 80 is a preferred surfactant.

Pharmaceutical compositions can include a preservative such as thiomersal or 2-phenoxyethanol. It is preferred that a composition should be substantially free from *(e.g.* <10µg/ml) mercurial material *e.g.* thiomersal-free. Compositions containing no mercury are more preferred. The inclusion of a preservative can be particularly useful when a composition includes an aluminum salt adjuvant because their insolubility means that the composition is generally a suspension having a cloudy appearance which can mask contaminating bacterial growth. A preservative is also particularly useful if a composition is to be used more than once *e.g.* in a multidose vial. Often, however, a pharmaceutical composition can be preservative-free.

Pharmaceutical compositions can have an osmolality of from 200 mOsm/kg and 400 mOsm/kg, *e.g.* from 240-360 mOsm/kg, or from 290-310 mOsm/kg.

Pharmaceutical compositions typically have a pH from 5.0 to 9.5 *e.g.* from 5.0 to 8.0 or from 6.0 to 8.0.

Pharmaceutical compositions are preferably non-pyrogenic *e.g.* containing <1 EU (endotoxin unit, a standard measure) per dose, and preferably <0.1 EU per dose.

Pharmaceutical compositions may have an osmolality from 200-400 mOsm/kg *e.g.* from 240-360 mOsm/kg, or from 280-320 mOsm/kg.

Pharmaceutical compositions are preferably gluten free.

Pharmaceutical compositions are suitable for administration to animal (and, in particular, human) patients, and thus include both human and veterinary uses.

Pharmaceutical compositions may be prepared in unit dose form. In some embodiments a unit dose may have a volume of from 0.1-1.0ml *e.g.* about 0.25mL or preferably about 0.5ml. Such volumes are ideal for injection in humans.

### Conjugate levels

A pharmaceutical composition can include multiple immunogenic conjugates. Currently licensed meningococcal conjugate vaccines include capsular saccharides from 4 different serogroups, and licensed pneumococcal conjugate vaccines include capsular saccharides from 7, 10, or 13 different serotypes. Thus a composition of the invention can include, for instance, from 3 to 50 different conjugates (*e.g.* 14, 15, 20, 21, 24, 25, or more). For example, each of these conjugates can include a capsular saccharide from a different serotype or serogroup of the same bacterial species (*e.g.* multiple meningococcal serogroups or multiple pneumococcal serotypes).

Where a pharmaceutical composition includes n different immunogenic conjugates, the total amount of carrier polypeptide in those *n* conjugates can be less than or equal to *3n* µg per dose. In other words, the average amount of carrier polypeptide per conjugate is less than 3µg. The total amount can, for instance, be from *n-2.5n* µg per dose.

Where a pharmaceutical composition includes n different immunogenic conjugates, the total amount of saccharide antigen in those *n* conjugates can be less than or equal to *4.4n* µg per dose. In other words, the average amount of saccharide per conjugate is less than 4.4µg. The total amount can, for instance, be from *0.4n-4.4n* µg per dose *e.g.* from 1.1*n*-2.2*n.*

Where a pharmaceutical composition includes *n* different immunogenic conjugates, the total concentration of carrier polypeptide for those *n* conjugates can be less than or equal to *6n* µg/mL. In other words, the average concentration of carrier polypeptide per conjugate is less than 6µg/mL. The total concentration can, for instance, be from *n-4n* µg/mL.

Where a pharmaceutical composition includes *n* different immunogenic conjugates, the total concentration of saccharide antigen for those *n* conjugates can be less than or equal to *8.8n* µg/mL. In other words, the average concentration of saccharide per conjugate is less than 8.8 µg/mL. The total concentration can, for instance, be from 0.8*n*-8.8*n* µg/mL *e.g.* from *2.2n-*4.4n µg/mL.

In some embodiments, the total amount of conjugated carrier polypeptide in a unit dose of a multivalent pharmaceutical composition of the invention can be from 4-128µg *e.g.* from 8-64µg, or from 16-48µg. The concentration of conjugated carrier polypeptide in a multivalent pharmaceutical composition of the invention can be from 8-256µg/mL *e*.*g*. from 16-128µg/mL, or from 32-96µg/mL.

In some embodiments, the total amount of conjugated saccharide antigen in a unit dose of a multivalent pharmaceutical composition of the invention can be from 10-120µg *e.g.* from 20-90µg, or from 30-60µg. The concentration of conjugated saccharide antigen in a multivalent pharmaceutical composition of the invention can be from 20-240µg/mL *e.g.* from 40-180µg/mL, or from 60-120µg/mL.

### Unconjugated components

As noted above, a pharmaceutical composition can include multiple immunogenic conjugates *e.g.* from 3 to 50 different conjugates *(e.g.* 14, 15, 20, 21, 24, 25, or more). For example, each of these conjugates can include a capsular saccharide from a different serotype or serogroup of the same bacterial species.

In some embodiments, the composition is free from the conjugates' carrier polypeptide(s) in unconjugated form. In other embodiments, there is a low level of unconjugated carrier polypeptide(s), provided that the mass of the unconjugated carrier polypeptide(s) in the composition is <10% *(e.g.* <5% or <2%) of the mass of the carrier polypeptide(s) in the *n* immunogenic conjugates of the composition as a whole.

In some embodiments, the composition is free from the conjugates' saccharides in unconjugated form. In other embodiments, there is a low level of unconjugated saccharide, provided that the mass of the unconjugated saccharide in the composition is <10% *(e.g.* <5% or <2%) of the total mass of saccharides in the n immunogenic conjugates of the composition.

### Containers, delivery devices, etc.

Pharmaceutical compositions including immunogenic conjugates can be packaged within sterile containers, delivery devices, *etc.* Sterility can be maintained by hermetically sealing a container to make it airtight. Suitable containers include, but are not limited to, vials, syringes, nebulisers, sprayers, inhalers, dermal patches, *etc.* Vials and syringes are preferred.

Immunogenic compositions are often contained within vials. The vial is preferably made of a plastic material or, preferably, of glass. A vial is sealed after being filled, and the seal can be broken at the point of use. The vial is preferably sterilized before a composition is added to it, and is then sealed. To avoid problems with latex-sensitive patients, vials can be sealed with a latex-free stopper, and the absence of latex in all packaging material is preferred. The vial ideally includes a single unit dose of a composition, but it may sometimes include more than one dose (a 'multidose' vial) *e.g.* 10 doses. Preferred vials are made of colorless glass.

A vial can have a cap *(e.g.* a Luer lock) adapted such that a syringe can be inserted into the cap to facilitate transfer of material between vial and syringe (in both directions). After removal of the syringe from the vial, a needle can then be attached and the composition can be administered to a subject. The cap is preferably located inside a seal or cover, such that the seal or cover has to be removed before the cap can be accessed. A vial may have a cap that permits aseptic removal of its contents, particularly for multidose vials.

Compositions can be contained within delivery devices, ready for administration to a subject. The composition can be transferred into the delivery device at the point of use *(e.g.* from a vial), or it can be put into the delivery device at the manufacturing stage *(e.g.* in the form of a pre-filled syringe).

A syringe used with the invention may be made of glass or of a plastic (*e.g.* made of a cyclo-olefin polymer or a cyclo-olefin co-polymer). A syringe (particularly a glass syringe) may be a siliconized syringe. Non-siliconized syringes can also be used *e.g.* using the i-Coating^{™} system from Terumo, which is available in their PLAJEX^{™} syringes, or using Daikyo CZ^{™} syringes having an ethylene-tetrafluoroethylene (ETFE) copolymer, or using a TriboGlide^{™} syringe having a perfluoropolyether (PFPE). Carbon films may be used instead of siliconization (*e.g.* see JP2001190665). Silicone-free syringes are also disclosed in JP2011212183. A non-siliconized syringe including a plunger stopper as disclosed in EP-A-0375778 may be used *i.e.* where the stopper has a thermoplastic elastomer layer which is covered at least in part by a thermoplastic resin layer of low dynamic friction coefficient.

Where a composition is contained within a syringe, the syringe may have a needle attached to it, for injection of the syringe's contents into a subject or into a container. The syringe may be supplied with a needle already attached. If a needle is not attached, a separate needle may be supplied with the syringe for assembly and use, or a needle can be separately sourced. Such a needle should be sterile at the time of use, and may be sheathed. Safety needles can be used. 1-inch 23-gauge, 1-inch 25-gauge and 5/8-inch 25-gauge needles are typical. Needles ½-1½ inch, 22-25 gauge, can be used. If the syringe and needle are packaged separately then the needle is preferably fitted with a butyl rubber shield.

Syringes may be provided with peel-off labels on which a lot number and expiration date of the contents may be printed, to facilitate record keeping. A plunger in a syringe can have a stopper to prevent the plunger from being accidentally removed during aspiration. The syringes may have a latex rubber cap and/or plunger, but latex-free rubbers can be used *eg.* latex-free chlorobutyl rubber or latex-free isoprene bromobutyl rubber. The syringe will generally have a tip cap to seal the tip prior to attachment of a needle, and the tip cap is preferably made of a butyl rubber *e.g.* latex-free isoprene bromobutyl rubber. Useful syringes are, for instance, those marketed under the trade name "Tip-Lok"^{™}.

Containers may be marked to show a half-dose volume *e.g.* to facilitate delivery to children. For instance, a syringe containing a 0.5ml dose may have a mark showing a 0.25ml volume. The syringe itself may have a volume larger than the dose *e.g.* 1 ml syringe can be used to contain a 0.5ml dose of a pharmaceutical composition. Disposable or pre-filled syringes usually contain a single dose of vaccine.

Where a glass container (*e.g.* a syringe or a vial) is used, then it is preferably made from a borosilicate glass rather than from a soda lime glass.

A container may be packaged *(e.g.* in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.* Multiple containers may be packaged together *e.g.* in the same box.

Pharmaceutical compositions can be presented in unit dose form, with a single dose per container (*e.g.* per syringe or per vial). Rather than manufacture each unit dose individually, a bulk composition is prepared and unit doses are extracted and individually packaged within their containers. Thus, for instance, a plurality of unit doses are extracted from the bulk and each unit dose is placed into a separate container *e.g.* into a syringe or a vial.

### Raising immune responses

An immunogenic conjugate can be administered to a mammalian subject to elicit a protective immune response against the antigen in that conjugate. They will be administered in the form of a pharmaceutical composition. The composition can include multiple immunogenic conjugates as described elsewhere herein, so protective immune responses can be elicited against many antigens simultaneously.

Thus we provide a method of eliciting a protective antibody response to one or more antigen(s) in a mammalian subject by administering to the subject a conjugate of the antigen(s).

We also provide a conjugate as disclosed herein, for use in eliciting a protective antibody response.

We also provide the use of a conjugate as disclosed herein in the manufacture of a medicament for eliciting a protective antibody response.

We also provide (i) a method of eliciting a protective antibody response to multiple antigen(s) in a mammalian subject by administering to the subject a multivalent composition of the invention, (ii) a multivalent composition of the invention for use in eliciting a protective antibody response, and (iii) the use of multiple conjugates as disclosed herein in the manufacture of a multivalent pharmaceutical composition for eliciting a protective antibody response against multiple antigens.

The ability to elicit a protective immune response means that the conjugates can be used, for example, to provide active immunization for the prevention of invasive disease caused by *S.pneumoniae,* for the prevention of otitis media caused by *Spneumoniae,* for the prevention of pneumonia caused by *Spneumoniae,* for active immunization of subjects at risk of exposure to *N.meningitidis* to prevent invasive disease, *etc.*

Pharmaceutical compositions can be prepared in various forms. For example, the compositions may be prepared as injectables *e.g.* as liquid solutions or suspensions. Injectables for intramuscular administration are typical. An injection volume of about 0.5ml is preferred for humans. Thus a preferred unit dose volume is about 0.5ml. Administration by intramuscular injection is typical *e.g.* in anterolateral aspect of the thigh in infants, or the deltoid muscle of the upper arm in toddlers, children and adults.

Conjugates will typically be administered according to a multiple dose schedule. Multiple doses may be used in a primary immunization schedule and/or in a booster immunization schedule. Administration of more than one dose (typically two doses) is particularly useful in immunologically naive patients. Multiple doses will typically be administered at least 1 week apart (*e.g.* about 2 weeks, about 3 weeks, about 4 weeks, about 6 weeks, about 8 weeks, about 10 weeks, about 12 weeks, *etc.).*

### General

The term "comprising" encompasses "including" as well as "consisting" e.g. a composition "comprising" X may consist exclusively of X or may include something additional *e.g.* X + Y.

The term "about" in relation to a numerical value *x* is optional and means, for example, *x*±10%.

The word "substantially" does not exclude "completely" *e.g.* a composition which is "substantially free" from Y may be completely free from Y. Where necessary, the word "substantially" may be omitted from the definition of the invention.

The term "sequence identity" in the context of two amino acid sequences refers to two sequences that are the same or have a specified percentage of amino acid residues that are the same, when compared and aligned for maximum correspondence over a comparison window, as measured using a sequence comparison algorithm (*e.g.,* BLASTP). The percent identity is determined over the full-length reference sequence disclosed herein, such as the reference sequence set forth in SEQ ID NO:1 or 2. The method for calculating the sequence identity as provided herein is the BLASTP program having its defaults set at a wordlength (W) of 3, an expectation (E) of 10, and the BLOSUM62 scoring matrix (see, *e.g.,* Henikoff & Henikoff, 1989, Proc Natl Acad Sci USA 89:10915). See e.g., the BLAST alignment tool available on the WWW at *blast.ncbi.nlm.nih.gov*/*Blast.cgi* or elsewhere.

The term "lower alkyl" as used herein, and unless otherwise specified, refers to a saturated straight or branched hydrocarbon having one to six carbon atoms, *i.e.,* C₁ to C₆ alkyl. In certain embodiments, the lower alkyl group is a primary, secondary, or tertiary hydrocarbon. The term includes both substituted and unsubstituted moieties. See also US-2014/0066598. The term "lower alkylene" refers to an alkylene radical of a lower alkyl.

Unless defined otherwise, all technical and scientific terms used herein have the commonly understood meaning. Practitioners are particularly directed to Green & Sambrook (eds.) Molecular Cloning: A Laboratory Manual, 4th ed., Cold Spring Harbor Laboratory Press, Cold Spring Harbor, N.Y. (2012), and Ausubel, F. M., et al., Current Protocols in Molecular Biology (Supplement 99), John Wiley & Sons, New York (2012), and Plotkin, S.A., Orenstein, W.A., & Offit, P.A., Vaccines, 6th ed, Elsevier, London (2013).

Methods for cell-free synthesis are described in Spirin & Swartz (2008) Cell-free Protein Synthesis, Wiley-VCH, Weinheim, Germany. Methods for incorporation of non-natural amino acids into proteins using cell-free synthesis are described in Shimizu et al. (2006) FEBS Journal, 273, 4133-4140 and also in Chong (2014) Curr Protoc Mol Biol. 108:16.30.1-11.

In some embodiments, the invention does not encompass a composition in which SEQ ID NO: 3 is used as the carrier polypeptide for conjugates from each of 24 pneumococcal serotypes 1, 2, 3, 4, 5, 6A, 6B, 7F, 8, 9N, 9V, 10A, 11A, 12F, 14, 15B, 17F, 18C, 19A, 19F, 20, 22F, 23F, and 33F (as exemplified in WO2018/126229). More generally, in some embodiments, the invention does not encompass a composition in which SEQ ID NO: 3 is used as the carrier polypeptide for each conjugate in a multivalent composition.

### ENUMERATED EMBODIMENTS

Embodiment I-1. A sterile container containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein.

Embodiment I-2. A hermetically sealed container containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein. Suitable containers for hermetic sealing include e.g. a vial. The contents are preferably sterile at the point of hermetic sealing.

Embodiment I-3. The container of embodiment I-1 or I-2, which is a sterile glass container such as a vial.

Embodiment I-4. A delivery device containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein.

Embodiment I-5. The container of embodiment I-1 or I-2, or the delivery device of embodiment I-4, which is a syringe.

Embodiment I-6. A pharmaceutical composition comprising two or more different immunogenic conjugates and an aluminum salt adjuvant, wherein: (i) each immunogenic conjugate comprises a carrier polypeptide and a saccharide antigen, wherein the saccharide antigen is covalently bonded to the carrier polypeptide via a non-natural amino acid residue therein; and (ii) the aluminum salt adjuvant is an aluminum hydroxide or aluminum phosphate adjuvant.

Embodiment I-7. A pharmaceutical composition comprising two or more different immunogenic conjugates, and an aluminum phosphate adjuvant, wherein: (i) each immunogenic conjugate comprises a carrier polypeptide and a saccharide antigen, wherein the saccharide antigen is covalently bonded to the carrier polypeptide via a non-natural amino acid residue therein; and (ii) the concentration of aluminum ions in the composition is ≤2.5mg/mL.

Embodiment I-8. A pharmaceutical composition comprising two or more different immunogenic conjugates, wherein: (i) each immunogenic conjugate comprises a carrier polypeptide and a saccharide antigen, wherein the saccharide antigen is covalently bonded to the carrier polypeptide via a non-natural amino acid residue therein; and (ii) the volume of the pharmaceutical composition is from 0.25-1.25mL.

Embodiment I-9. A pharmaceutical composition comprising two or more different immunogenic conjugates and a preservative, wherein each immunogenic conjugate comprises a carrier polypeptide and a saccharide antigen, wherein the saccharide antigen is covalently bonded to the carrier polypeptide via a non-natural amino acid residue therein.

Embodiment I-10. A preservative-free pharmaceutical composition comprising two or more different immunogenic conjugates, wherein each immunogenic conjugate comprises a carrier polypeptide and a saccharide antigen, wherein the saccharide antigen is covalently bonded to the carrier polypeptide via a non-natural amino acid residue therein.

Embodiment I-11. A pharmaceutical composition comprising two or more different immunogenic conjugates, wherein: (i) each immunogenic conjugate comprises a carrier polypeptide and a saccharide antigen, wherein the saccharide antigen is covalently bonded to the carrier polypeptide via a non-natural amino acid residue therein; (ii) the composition has an osmolality from 200-400 mOsm/kg.

Embodiment I-12. A pharmaceutical composition comprising two or more different immunogenic conjugates and at least one excipient wherein: (i) each immunogenic conjugate comprises a carrier polypeptide and a saccharide antigen, wherein the saccharide antigen is covalently bonded to the carrier polypeptide via a non-natural amino acid residue therein; and (ii) the at least one excipient is selected from the group consisting of sodium chloride, succinic acid, and polysorbate 80.

Embodiment I-13. A pharmaceutical composition comprising n different immunogenic conjugates wherein:
(i) each of the *n* immunogenic conjugates comprises a carrier polypeptide and a saccharide antigen, wherein the saccharide antigen is covalently bonded to the carrier polypeptide via a non-natural amino acid residue therein;
(ii) n is an integer from 3 to 50; and
(iii) the total amount of carrier polypeptide in the n immunogenic conjugates is less than or equal to *3n* µg per dose;
(iv) the total concentration of carrier polypeptide in the n immunogenic conjugates is less than or equal to 6*n* µg/ml;
(v) the total amount of saccharide antigen in the n immunogenic conjugates is less than or equal to *3n* µg per dose;
(vi) the total concentration of saccharide antigen in the n immunogenic conjugates is less than or equal to 6n µg/mL;
(vii) the average amount of carrier polypeptide per conjugate is from 1-4 µg per dose;
(viii) the average concentration of carrier polypeptide per conjugate is from 2-8 µg/mL;
(ix) the average amount of saccharide antigen per conjugate is from 1-4 µg per dose;
(x) the average concentration of saccharide antigen per conjugate is from 2-8 µg/mL;
(xi) the composition is free from the carrier polypeptide(s) in unconjugated form;
(xii) the composition contains the carrier polypeptide(s) in unconjugated form, wherein the mass of the carrier polypeptide(s) in unconjugated form in the composition is <10% of the mass of that carrier polypeptide in the *n* immunogenic conjugates;
(xiii) the composition is free from the saccharide antigens in unconjugated form; and/or
(xiv) the composition contains at least one of the saccharide antigens in unconjugated form, wherein the total mass of the saccharide antigens in unconjugated form in the composition is <10% of the total mass of the saccharide antigens in the *n* immunogenic conjugates.

Embodiment I-14. A process for preparing a plurality of unit doses of a pharmaceutical composition, wherein (i) the pharmaceutical composition comprises an immunogenic conjugate comprising a carrier polypeptide and a saccharide antigen, wherein the saccharide antigen is covalently bonded to the carrier polypeptide via a non-natural amino acid residue therein, and (ii) the process comprises steps of preparing a bulk composition comprising the immunogenic conjugate and packaging individual unit doses from the bulk composition into a plurality of individual containers.

Embodiment I-15. A process for preparing a pharmaceutical composition, wherein the pharmaceutical composition comprises two or more different immunogenic conjugates and an aluminum salt adjuvant, wherein (i) each of the immunogenic conjugates comprises a carrier polypeptide and a saccharide antigen, and (ii) the saccharide antigen is covalently bonded to the carrier polypeptide via a non-natural amino acid residue therein; and the process comprises either (A) steps of separately adsorbing each of the immunogenic conjugates to an aluminum salt adjuvant then mixing individual adsorbed conjugates together or (B) sequentially adsorbing each of the immunogenic conjugates to the aluminum salt adjuvant.

Embodiment I-16. A carrier polypeptide comprising an amino acid sequence which (i) has at least 80% sequence identity to SEQ ID NO: 1; (ii) is free from an Arg-Arg dipeptide sequence; and (iii) includes at least one nnAA residue.

Embodiment I-17. A carrier polypeptide comprising an amino acid sequence which (i) has at least 80% sequence identity to SEQ ID NO: 1 and (ii) includes a nnAA substitution at one or more of the following amino acid residues (numbered according to SEQ ID NO: 1): Asp-211; Asp-295; Asp-352; Asp-392; Asp-465; Asp-467; Asp 507; Asp 519; Asn 296; Asn 359; Asn 399; Asn 481; Asn 486; Asn 502; Asn 524; Glu 240; Glu 248; Glu 249; Glu 256; Glu 259; Glu 292; Glu 362; Gln 252; Gln 287; Lys 212; Lys 218; Lys 221; Lys 229; Lys 236; Lys 264; Lys 299; Lys 385; Lys 456; Lys 474; Lys 498; Lys 516; Lys 522; Lys 534; Arg 377; Arg 407; Arg 455; Arg 460; Arg 462; Arg 472; Arg 493; Ser 198; Ser 200; Ser 231; Ser 233; Ser 239; Ser 261; Ser 374; Ser 381; Ser 297; Ser 397; Ser 451; Ser 475; Ser 494; Ser 495; Ser 496; Ser 501; Ser 505; Thr 253; Thr 265; Thr 267; Thr 269; Thr 293; Thr 386; Thr 400; Thr 408; Thr-469; and/or Thr 517.

Embodiment I-18. The carrier polypeptide of embodiment I-16 or I-17, wherein Arg-193 of SEQ ID NO: 1 is substituted with a different amino acid, such as Asn.

Embodiment I-19. An immunogenic conjugate comprising the carrier polypeptide of embodiment 1-16 or 1-17 or 1-18, conjugated via a nnAA residue therein to an antigen.

Embodiment I-20. An immunogenic conjugate comprising a carrier polypeptide and a saccharide antigen, wherein (i) the carrier polypeptide comprises amino acid sequence SEQ ID NO: 4; and (ii) the saccharide antigen is covalently bonded to the carrier polypeptide via at least one nnAA residue within SEQ ID NO: .

Embodiment I-21. A pharmaceutical composition comprising two or more different immunogenic conjugates according to embodiment I-20.

Embodiment I-22. The container, device, composition, process, polypeptide, or conjugate of any preceding embodiment, wherein the carrier polypeptide comprises 4 to 9 nnAA residues.

Embodiment I-23. The container, device, composition, process, polypeptide, or conjugate of any preceding embodiment, wherein at least one nnAA is substituted for a lysine in the carrier polypeptide's native sequence.

Embodiment I-24. The container, device, composition, process, polypeptide, or conjugate of any preceding embodiment, wherein the carrier polypeptide has at least 90% sequence identity to SEQ ID NO:1.

Embodiment I-25. The container, device, composition, process, polypeptide, or conjugate of embodiment I-24, wherein at least one nnAA is substituted for K24, K33, K37, K39, K212, K214, K227, K244, K264, K385, K522 and/or K526 in SEQ ID NO: 1 or 2.

Embodiment I-26. The container, device, composition, process, polypeptide, or conjugate of any preceding embodiment, wherein the carrier polypeptide comprises amino acid sequence SEQ ID NO: 14.

Embodiment I-27. The container, device, composition, process, polypeptide, or conjugate of any preceding embodiment, wherein the nnAA is 2-amino-3-(4-(azidomethyl)phenyl)propanoic acid.

Embodiment I-28. The container, device, composition, process, polypeptide, or conjugate of any preceding embodiment, wherein the antigen has an alkyne group which is conjugated to the nnAA via an azido group.

Embodiment I-29. The container, device, composition, process, polypeptide, or conjugate of any preceding embodiment, wherein the antigen is a bacterial capsular saccharide; for example, a capsular saccharide from a bacterium selected from the group consisting of *Streptococcus pneumoniae, Neisseria meningitidis, Haemophilus influenzae, Streptococcus pyogenes, Streptococcus agalactiae,* and *Porphyromonas gingivalis.*

Embodiment I-30. The container, device, composition, process, polypeptide, or conjugate of any preceding embodiment, wherein the antigen is a capsular saccharide of a *S.pneumoniae* serotype selected from the group consisting of 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F.

Embodiment 1-31. The container, device, composition, process, polypeptide, or conjugate of any preceding embodiment, wherein ratio of saccharide to carrier polypeptide (w/w) in the conjugate(s) is greater than 1.

Embodiment I-32. The container, device, composition, process, polypeptide, or conjugate of any preceding embodiment, wherein the carrier polypeptide includes 3 or more nnAA residues and the conjugate has a molecular weight of at least 500kDa.

Embodiment I-33. The container, device, composition, process, polypeptide, or conjugate of any preceding embodiment, wherein the conjugate has a molecular weight between 900kDa and SMDa.

Embodiment I-34. The container, device, composition or process, of any one of embodiments 1-1 to 1-15 or embodiments I-21 to 1-33, wherein pharmaceutical composition comprises:
conjugates of capsular saccharides from 2 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
conjugates of capsular saccharides from 14 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
conjugates of capsular saccharides from 15 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
conjugates of capsular saccharides from 20 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
conjugates of capsular saccharides from 21 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
conjugates of capsular saccharides from 24 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
conjugates of capsular saccharides from 25 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
conjugates of capsular saccharides from 4 or more different meningococcal serogroups selected from the group consisting of serogroups A, C, W135, X, and Y; or
conjugates of capsular saccharides from 2 or more different *P.gingivals* serotypes selected from the group consisting of serotypes K1, K2, K3, K4, K5, and K6.

Embodiment 1-35. A method of eliciting an immunoprotective antibody response to an antigen in a subject, comprising administering to the subject a pharmaceutical composition of any one of embodiments I-6 to I-13 or embodiments I-21 to I-34, or an immunogeinc conjugate according to any of embodiments I-19 to I-33, in an excipient suitable for parenteral administration.

### EXAMPLES

The invention is illustrated in the following examples. The materials, methods, and examples are illustrative only and not intended to be limiting. Numerous variations, changes, and substitutions will occur to those skilled in the art without departing from the invention. The examples are carried out using well known and routine techniques to those of skill in the art, except where otherwise described in detail.

### Examples from WO2018/126229

The examples of WO2018/126229 describe in full detail the synthesis of single-site eCRM moieties (e.g. K11TAG). These were expressed in a cell-free protein synthesis (CFPS) extract, and pAMF was incorporated in place of natural Lys.

Variants of CRM containing multiple nnAA per polypeptide were also expressed, with various different numbers of Lys-pAMF substitutions per protein. In general it was found that higher numbers of substitutions gave carriers which led to higher MW conjugates but the carriers had lower solubility. Carriers with six pAMF residues generally provided both good solubility (>>50mg/mL) and immunogenicity. The high solubility was surprising because replacement of charged Lys residues in the native sequence with hydrophobic pAMF residues increased the hydrophobicity of CRM197, which is a protein whose hydrophobicity has already been reported to affect its solubility. Thus it was shown that it is possible to maintain the same attachment sites which have been used in known CRM197 conjugates (namely Lys residues) without causing insolubility when the charged residues are lost.

A particularly useful set of 6 Lys-pAMF substitutions was seen using K34, K213, K245, K265, K386 and K527 (numbered according to SEQ ID NO: 3). This combination of sites for pAMF substitution is surprisingly effective, in particular because individual substitutions at positions K245 and K527 led to relatively poor levels of expression.

This set of six substitutions can be combined with disruption of the Arg-Arg dipeptide at residues 192-193 of SEQ ID NO: 1 (RR->RN) to provide SEQ ID NO: 4, where each X is pAMF.

The examples of WO2018/126229 further describe general protocols for saccharide activation with sodium meta-periodate, for periodate-oxidized polysaccharide derivatization with DBCO, for saccharide activation with CDAP, and for conjugation of saccharide-DBCO with eCRM. See also U.S. Serial No. 62/693,978, previously incorporated by reference

### Multivalent immunogenic composition

A combination of conjugates for each of 24 pneumococcal serotypes 1, 2, 3, 4, 5, 6A, 6B, 7F, 8, 9N, 9V, 10A, 11A, 12F, 14, 15B, 17F, 18C, 19A, 19F, 20, 22F, 23F, and 33F was prepared using CRM197 derivative SEQ ID NO: 4 (with X=pAMF) as the carrier polypeptide in each conjugate. The immunogenicity of this multivalent composition was confirmed using a 3-dose schedule in groups of 7 rabbits, by intramuscular injection of 0.25mL vaccine. Each dose included 24µg saccharide (1µg per serotype), giving a concentration of 96µg/mL.

A combination of conjugates for each of 32 pneumococcal serotypes 1, 2, 3, 4, 5, 6A, 6B, 6C, 7C, 7F, 8, 9N, 9V, 10A, 11A, 12F, 14, 15A, 15B, 16F, 17F, 18C, 19A, 19F, 20, 22F, 23A, 23B, 23F, 31, 33F, and 35B was then prepared and immunogenicity confirmed in a similar manner.

For comparison purposes, the 13-valent Prevnar^{™} conjugate vaccine was also tested, along with a 24-valent unconjugated vaccine made of the 23-valent Pneumovax^{™} vaccine supplemented by unconjugated serotype 6A polysaccharide. These three compositions had equivalent polysaccharide doses per serotype (except for 6B, where Prevnar^{™} includes a double dosage), which involved diluting the Prevnar^{™} and Pneumovax^{™}. All three compositions included aluminum phosphate adjuvant (60µg Al⁺⁺⁺ per dose), which involved adding the adjuvant to Pneumovax^{™}. The compositions were preservative-free.

The 24-valent conjugate composition included a lower amount of carrier polypeptide than in the approved Prevnar-13^{™} vaccine, even though it also includes capsular saccharides from 11 additional serotypes. The overall weight ratio of capsular saccharide to carrier polypeptide in the 24-valent conjugate composition was about double that seen in Prevnar^{™}.

IgG and OPA responses were measured in the rabbits. After the third dose both of these responses were much greater in rabbits which received the two conjugated vaccines than in those which received the unconjugated vaccine. Moreover, IgG and OPA responses using the 24-valent composition were comparable to those achieved using Prevnar^{™} in the 13 serotypes covered by the approved vaccine, but were in addition superior against the 11 serotypes which are not included in Prevnar^{™}. Surprisingly, there was no evidence of carrier induced epitopic suppression using the 24-valent composition.

FIG.1 provides the geometric mean titer for each of the 32 serotypes in the 32-valent conjugate composition relative to the polysaccharide/alum formulation and Prevnar-13^{™}.

The multivalent conjugate composition can usefully be packaged into a pre-filled sterile syringe so that it can be easily distributed in unit dose form, and can then be administered at the point of use without needing to transfer the contents of a vial into a syringe for injection *etc.*

### Substitutable positions in CRM197

Building on the work disclosed in WO2018/126229, a variety of Asp, Asn, Glu, Gln, Lys, Arg, Ser and Thr residues within the native CRM197 sequence (SEQ ID NO: 1) were individually replaced with pAMF by mutating their codons to TAG and expressing the protein at 25°C in a cell-free system in which this codon is recognized by a tRNA which incorporates the nnAA. The mutant polypeptides were expressed with a N-terminus methionine and with a downstream hexahistidine tag attached via a Gly-Ser-Gly tripeptide linker. Residues within Asn270-Ile289, Ala320-Glu349 and Phe410-His-449 were avoided because of the recognized T-cell epitopes in these regions (see above).

Expression efficiency was assessed by checking ¹⁴C-Leu incorporation into the mutant proteins, looking both at total protein and soluble protein. In general, mutations in the catalytic domain of CRM197 led to reduced expression levels relative to the unmodified CRM197 sequence, and the mutants with the best expression levels generally involved substitutions downstream of Arg-193, which can be used to delineate the end of the catalytic domain.

The best 72 mutants showed increased expression levels of both total and soluble proteins and had substitutions at the following residues, numbered according to SEQ ID NO: 1: Ser-198, Ser-200, Asp-211, Lys-212, Lys-218, Lys-221, Lys-229, Ser-231, Ser-233, Lys-236, Ser-239, Glu-240, Glu-248, Glu-249, Gln-252, Thr-253, Glu-256, Glu-259, Ser-261, Lys-264, Thr-265, Thr-267, Thr-269, Gln-287, Glu-292, Thr-293, Asp-295, Asn-296, Ser-297, Lys-299, Asp-352, Asn-359, Glu-362, Ser-374, Arg-377, Ser-381, Lys-385, Thr-386, Asp-392, Ser-397, Asn-399, Thr-400, Arg-407, Thr-408, Ser-451, Arg-455, Lys-456, Arg-460, Arg-462, Asp-465, Asp-467, Thr-469, Arg-472, Lys-474, Ser-475, Asn-481, Asn-486, Arg-493, Ser-494, Ser-495, Ser-496, Lys-498, Ser-501, Asn-502, Ser-505, Asp-507, Lys-516, Thr-517, Asp-519, Lys-522, Asn-524, and Lys-534.

The embodiments described herein are provided by way of example only, and various alternatives to the embodiments are not excluded in practising the embodiments described herein.

### EMBODIMENTS

1. A sterile container containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein.
2. A hermetically sealed container containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein. Suitable containers for hermetic sealing include e.g. a vial. The contents are preferably sterile at the point of hermetic sealing.
3. The container of embodiment 1 or embodiment 2, which is a sterile glass container such as a vial.
4. A delivery device containing a pharmaceutical composition which comprises an immunogenic conjugate comprising a carrier polypeptide and a saccharide antigen, wherein the saccharide antigen is covalently bonded to the carrier polypeptide via a non-natural amino acid residue therein.
5. The container of embodiment 1 or embodiment 2, or the delivery device of embodiment 4, which is a syringe.
6. A pharmaceutical composition comprising two or more different immunogenic conjugates and an aluminum salt adjuvant, wherein: (i) each immunogenic conjugate comprises a carrier polypeptide and a saccharide antigen, wherein the saccharide antigen is covalently bonded to the carrier polypeptide via a non-natural amino acid residue therein; and (ii) the aluminum salt adjuvant is an aluminum hydroxide or aluminum phosphate adjuvant.
7. A pharmaceutical composition comprising two or more different immunogenic conjugates, and an aluminum phosphate adjuvant, wherein: (i) each immunogenic conjugate comprises a carrier polypeptide and a saccharide antigen, wherein the saccharide antigen is covalently bonded to the carrier polypeptide via a non-natural amino acid residue therein; and (ii) the concentration of aluminum ions in the composition is ≤2.5mg/mL.
8. A pharmaceutical composition comprising two or more different immunogenic conjugates, wherein: (i) each immunogenic conjugate comprises a carrier polypeptide and a saccharide antigen, wherein the saccharide antigen is covalently bonded to the carrier polypeptide via a non-natural amino acid residue therein; and (ii) the volume of the pharmaceutical composition is from 0.25-1.25mL.
9. A pharmaceutical composition comprising two or more different immunogenic conjugates and a preservative, wherein each immunogenic conjugate comprises a carrier polypeptide and a saccharide antigen, wherein the saccharide antigen is covalently bonded to the carrier polypeptide via a non-natural amino acid residue therein.
10. A preservative-free pharmaceutical composition comprising two or more different immunogenic conjugates, wherein each immunogenic conjugate comprises a carrier polypeptide and a saccharide antigen, wherein the saccharide antigen is covalently bonded to the carrier polypeptide via a non-natural amino acid residue therein.
11. A pharmaceutical composition comprising two or more different immunogenic conjugates, wherein: (i) each immunogenic conjugate comprises a carrier polypeptide and a saccharide antigen, wherein the saccharide antigen is covalently bonded to the carrier polypeptide via a non-natural amino acid residue therein; (ii) the composition has an osmolality from 200-400 mOsm/kg.
12. A pharmaceutical composition comprising two or more different immunogenic conjugates and at least one excipient wherein: (i) each immunogenic conjugate comprises a carrier polypeptide and a saccharide antigen, wherein the saccharide antigen is covalently bonded to the carrier polypeptide via a non-natural amino acid residue therein; and (ii) the at least one excipient is selected from the group consisting of sodium chloride, succinic acid, and polysorbate 80.
13. A pharmaceutical composition comprising *n* different immunogenic conjugates wherein:
   (i) each of the *n* immunogenic conjugates comprises a carrier polypeptide and a saccharide antigen, wherein the saccharide antigen is covalently bonded to the carrier polypeptide via a non-natural amino acid residue therein;
   (ii) n is an integer from 3 to 50; and
   (iii) the total amount of carrier polypeptide in the n immunogenic conjugates is less than or equal to *3n* µg per dose;
   (iv) the total concentration of carrier polypeptide in the n immunogenic conjugates is less than or equal to 6*n* µg/ml;
   (v) the total amount of saccharide antigen in the n immunogenic conjugates is less than or equal to *3n* µg per dose;
   (vi) the total concentration of saccharide antigen in the n immunogenic conjugates is less than or equal to 6*n* µg/mL;
   (vii) the average amount of carrier polypeptide per conjugate is from 1-4 µg per dose;
   (viii) the average concentration of carrier polypeptide per conjugate is from 2-8 µg/mL;
   (ix) the average amount of saccharide antigen per conjugate is from 1-4 µg per dose;
   (x) the average concentration of saccharide antigen per conjugate is from 2-8 µg/mL;
   (xi) the composition is free from the carrier polypeptide(s) in unconjugated form;
   (xii) the composition contains the carrier polypeptide(s) in unconjugated form, wherein the mass of the carrier polypeptide(s) in unconjugated form in the composition is <10% of the mass of that carrier polypeptide in the *n* immunogenic conjugates;
   (xiii) the composition is free from the saccharide antigens in unconjugated form; and/or
   (xiv) the composition contains at least one of the saccharide antigens in unconjugated form, wherein the total mass of the saccharide antigens in unconjugated form in the composition is <10% of the total mass of the saccharide antigens in the n immunogenic conjugates.
14. A process for preparing a plurality of unit doses of a pharmaceutical composition, wherein (i) the pharmaceutical composition comprises an immunogenic conjugate comprising a carrier polypeptide and a saccharide antigen, wherein the saccharide antigen is covalently bonded to the carrier polypeptide via a non-natural amino acid residue therein, and (ii) the process comprises steps of preparing a bulk composition comprising the immunogenic conjugate and packaging individual unit doses from the bulk composition into a plurality of individual containers.
15. A process for preparing a pharmaceutical composition, wherein the pharmaceutical composition comprises two or more different immunogenic conjugates and an aluminum salt adjuvant, wherein (i) each of the immunogenic conjugates comprises a carrier polypeptide and a saccharide antigen, and (ii) the saccharide antigen is covalently bonded to the carrier polypeptide via a non-natural amino acid residue therein; and the process comprises either (A) steps of separately adsorbing each of the immunogenic conjugates to an aluminum salt adjuvant then mixing individual adsorbed conjugates together or (B) sequentially adsorbing each of the immunogenic conjugates to the aluminum salt adjuvant.
16. A carrier polypeptide comprising an amino acid sequence which (i) has at least 80% sequence identity to SEQ ID NO: 1; (ii) is free from an Arg-Arg dipeptide sequence; and (iii) includes at least one nnAA residue.
17. A carrier polypeptide comprising an amino acid sequence which (i) has at least 80% sequence identity to SEQ ID NO: 1 and (ii) includes a nnAA substitution at one or more of the following amino acid residues (numbered according to SEQ ID NO: 1): Asp-211; Asp-295; Asp-352; Asp-392; Asp-465; Asp-467; Asp 507; Asp 519; Asn 296; Asn 359; Asn 399; Asn 481; Asn 486; Asn 502; Asn 524; Glu 240; Glu 248; Glu 249; Glu 256; Glu 259; Glu 292; Glu 362; Gln 252; Gln 287; Lys 212; Lys 218; Lys 221; Lys 229; Lys 236; Lys 264; Lys 299; Lys 385; Lys 456; Lys 474; Lys 498; Lys 516; Lys 522; Lys 534; Arg 377; Arg 407; Arg 455; Arg 460; Arg 462; Arg 472; Arg 493; Ser 198; Ser 200; Ser 231; Ser 233; Ser 239; Ser 261; Ser 374; Ser 381; Ser 297; Ser 397; Ser 451; Ser 475; Ser 494; Ser 495; Ser 496; Ser 501; Ser 505; Thr 253; Thr 265; Thr 267; Thr 269; Thr 293; Thr 386; Thr 400; Thr 408; Thr-469; and/or Thr 517.
18. The carrier polypeptide of embodiment 16 or embodiment 17, wherein Arg-193 of SEQ ID NO: 1 is substituted with a different amino acid, such as Asn.
19. An immunogenic conjugate comprising the carrier polypeptide of embodiment 16 or embodiment 17 or embodiment 18, conjugated via a nnAA residue therein to an antigen.
20. An immunogenic conjugate comprising a carrier polypeptide and a saccharide antigen, wherein (i) the carrier polypeptide comprises amino acid sequence SEQ ID NO: 4; and (ii) the saccharide antigen is covalently bonded to the carrier polypeptide via at least one nnAA residue within SEQ ID NO: 4.
21. A pharmaceutical composition comprising two or more different immunogenic conjugates according to embodiment 20.
22. The container, device, composition, process, polypeptide, or conjugate of any preceding embodiment, wherein the carrier polypeptide comprises 4 to 9 nnAA residues.
23. The container, device, composition, process, polypeptide, or conjugate of any preceding embodiment, wherein at least one nnAA is substituted for a lysine in the carrier polypeptide's native sequence.
24. The container, device, composition, process, polypeptide, or conjugate of any preceding embodiment, wherein the carrier polypeptide has at least 90% sequence identity to SEQ ID NO: 1.
25. The container, device, composition, process, polypeptide, or conjugate of embodiment 24, wherein at least one nnAA is substituted for K24, K33, K37, K39, K212, K214, K227, K244, K264, K385, K522 and/or K526 in SEQ ID NO:1 or 2.
26. The container, device, composition, process, polypeptide, or conjugate of any preceding embodiment, wherein the carrier polypeptide comprises amino acid sequence SEQ ID NO: 4.
27. The container, device, composition, process, polypeptide, or conjugate of any preceding embodiment, wherein the nnAA is 2-amino-3-(4-(azidomethyl)phenyl)propanoic acid.
28. The container, device, composition, process, polypeptide, or conjugate of any preceding embodiment, wherein the antigen has an alkyne group which is conjugated to the nnAA via an azido group.
29. The container, device, composition, process, polypeptide, or conjugate of any preceding embodiment, wherein the antigen is a bacterial capsular saccharide; for example, a capsular saccharide from a bacterium selected from the group consisting of *Streptococcus pneumoniae, Neisseria meningitidis, Haemophilus influenzae, Streptococcus pyogenes, Streptococcus agalactiae,* and *Porphyromonas gingivalis.*
30. The container, device, composition, process, polypeptide, or conjugate of any preceding embodiment, wherein the antigen is a capsular saccharide of a *S.pneumoniae* serotype selected from the group consisting of 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F.
31. The container, device, composition, process, polypeptide, or conjugate of any preceding embodiment, wherein ratio of saccharide to carrier polypeptide (w/w) in the conjugate(s) is greater than 1.
32. The container, device, composition, process, polypeptide, or conjugate of any preceding embodiment, wherein the carrier polypeptide includes 3 or more nnAA residues and the conjugate has a molecular weight of at least 500kDa.
33. The container, device, composition, process, polypeptide, or conjugate of any preceding embodiment, wherein the conjugate has a molecular weight between 900kDa and 5MDa.
34. The container, device, composition or process, of any one of embodiments 1-15 or 21-33, wherein pharmaceutical composition comprises:
   conjugates of capsular saccharides from 2 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
   conjugates of capsular saccharides from 14 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
   conjugates of capsular saccharides from 15 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
   conjugates of capsular saccharides from 20 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
   conjugates of capsular saccharides from 21 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
   conjugates of capsular saccharides from 24 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
   conjugates of capsular saccharides from 25 or more different pneumococcal serotypes selected from the group consisting of serotypes 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F;
   conjugates of capsular saccharides from 4 or more different meningococcal serogroups selected from the group consisting of serogroups A, C, W135, X, and Y; or
   conjugates of capsular saccharides from 2 or more different *P.gingivals* serotypes selected from the group consisting of serotypes K1, K2, K3, K4, K5, and K6.
35. The container, device, composition, process, polypeptide, or conjugate of any one of embodiments 30-34, wherein serotype 20 is serotype 20B.
36. The container, device, composition, process, polypeptide, or conjugate of any one of embodiments 30-34, wherein serotype 20 is serotype 20A.
37. A method of eliciting an immunoprotective antibody response to an antigen in a subject, comprising administering to the subject a pharmaceutical composition of any one of embodiments 6-13 or 21-34, or an immunogeinc conjugate according to any of embodiments 19-33, in an excipient suitable for parenteral administration.

## Claims

1. A pharmaceutical composition comprising two or more different immunogenic conjugates, wherein:
(i) each immunogenic conjugate comprises a carrier polypeptide and a saccharide antigen, wherein the saccharide antigen is covalently bonded to the carrier polypeptide via a non-natural amino acid residue therein; and
(ii) at least one of
a) the pharmaceutical composition further comprises an aluminum salt adjuvant that is an aluminum hydroxide or aluminum phosphate adjuvant;
b) the pharmaceutical composition further comprises an aluminum salt adjuvant wherein the concentration of aluminum ions in the composition is < 2.5 mg/mL;
c) the volume of the pharmaceutical composition is from 0.25 mL-1.25 mL;
d) the composition has an osmolality from 200-400 mOsm/kg;
e) the pharmaceutical composition further comprises at least one excipient selected from the group consisting of sodium chloride, succinic acid, and polysorbate 80.

2. The pharmaceutical composition of any previous claim, wherein the carrier polypeptides of the two or more different immunogenic conjugates comprise an amino acid sequence having at least 80% sequence identity to SEQ ID NO: 1.

3. The pharmaceutical composition of any previous claim, wherein the carrier polypeptides of the two or more different immunogenic conjugates are free from an Arg-Arg dipeptide sequence.

4. The pharmaceutical composition of any previous claim, wherein the non-natural amino acid is 2-amino-3-(4-(azidomethyl)phenyl)propanoic acid.

5. The pharmaceutical composition of any previous claim, wherein the non-natural amino acid residue is located at one of residues (numbered according to SEQ ID NO: 1): Asp-211; Asp-295; Asp-352; Asp-392; Asp-465; Asp-467; Asp 507; Asp 519; Asn 296; Asn 359; Asn 399; Asn 481; Asn 486; Asn 502; Asn 524; Glu 240; Glu 248; Glu 249; Glu 256; Glu 259; Glu 292; Glu 362; Gln 252; Gln 287; Lys 212; Lys 218; Lys 221; Lys 229; Lys 236; Lys 264; Lys 299; Lys 385; Lys 456; Lys 474; Lys 498; Lys 516; Lys 522; Lys 534; Arg 377; Arg 407; Arg 455; Arg 460; Arg 462; Arg 472; Arg 493; Ser 198; Ser 200; Ser 231; Ser 233; Ser 239; Ser 261; Ser 374; Ser 381; Ser 297; Ser 397; Ser 451; Ser 475; Ser 494; Ser 495; Ser 496; Ser 501; Ser 505; Thr 253; Thr 265; Thr 267; Thr 269; Thr 293; Thr 386; Thr 400; Thr 408; Thr-469; and/or Thr 517.

6. The pharmaceutical composition of any previous claim, wherein the non-natural amino acid is substituted for a lysine in the native sequences of the carrier polypeptides of the two or more different immunogenic conjugates.

7. The pharmaceutical composition of any previous claim, wherein the saccharide antigens of the two or more different immunogenic conjugates are bacterial capsular saccharides; for example, from a bacterium selected from the group consisting of Streptococcus pneumoniae, Neisseria meningitidis, Haemophilus influenzae, Streptococcus pyogenes, Streptococcus agalactiae, and Porphyromonas gingivalis.

8. The pharmaceutical composition of any previous claim, wherein the saccharide antigens of the two or more different immunogenic conjugates are capsular saccharides of a *S. pneumoniae* serotype, for example selected from the group consisting of 1, 2, 3, 4, 5, 6A, 6B, 7F, 8, 9V, 9N, 10A, 11A, 12F, 13, 14, 15B, 16, 17F, 18C, 19A, 19F, 20, 22F, 23F, 24F, 31, and 33F.

9. The pharmaceutical composition of any previous claim further comprising a preservative.

10. A method of eliciting an immunoprotective antibody response to an antigen in a subject, comprising administering to the subject the pharmaceutical composition of any previous claim.
